# EUROPEAN PATENT APPLICATION

(11) **EP 3 318 282 A1**
(43) Date of publication of application: **09.05.2018**
(21) Application number: 16818087.5
(22) Date of filing: 01.07.2016
(51) Int. Cl.: A61K 47/34, A61K 47/10, A61K 47/18, A61K 47/26

(54) **DRUG DELIVERY CARRIER, AND COMPOSITION CONTAINING SAME**

(30) Priority: 02.07.2015 US 201562187816 P
(71) Applicant: The University Of Tokyo, Tokyo 113-8654 (JP)
(72) Inventor: KATAOKA, Kazunori, Tokyo 113-8654 (JP); ANRAKU, Yasutaka, Tokyo 113-8654 (JP); HORI, Mao, Tokyo 113-8654 (JP)
(74) Representative: dompatent von Kreisler Selting Werner - Partnerschaft von Patent- und Rechtsanwälten mbB
(86) International application number: PCT/JP2016/069725
(87) International publication number: WO 2017/002979

(57) **Abstract**

The present invention provides a carrier containing a first block copolymer and a second block copolymer, wherein the first block copolymer is a block copolymer of a first non-charged segment and a first complex-forming segment, at least some proportion of said first block copolymer is modified by a first molecule, the second block copolymer is a block copolymer of a second non-charged segment and a second complex-forming segment, and wherein at least some proportion of said second block copolymer is modified by a second molecule, said first molecule is a GLUT1 ligand, and said second molecule is different from the first molecule.

## Description

### [Technical Field]

The present invention relates to a drug delivery carrier and a composition containing same. In addition, the present invention relates to a method of delivering a drug by using the carrier or composition.

### [Background Art]

It is known that a blood-brain barrier exists between the blood and the brain for restricting substance exchange. This is considered to be based on the formation of tight junction by brain vascular endothelial cells to produce an extremely narrow cell-cell gap as well as selective uptake and export of substances by the cells themselves.

The blood-brain barrier has high permeation selectivity and substances can hardly pass through the barrier except some substances (e.g., alcohol, caffeine, nicotine and glucose). This has made it difficult to treat brain diseases with therapeutic drugs for the brain, diagnose brain diseases with brain diagnostic agents or perform imaging of the brain with a contrast agent.

A technique for delivering an antibody to the brain by utilizing the property of glucose to pass the blood-brain barrier has been developed (patent document 1). However, in this technique, the antibody is merely glycosylated and the effect thereof is limited.

The present inventors developed a technique for efficiently delivering a vesicle such as micelle and the like encapsulating a drug to the brain by encapsulating the drug in the vesicle having an outer surface modified with glucose, administering same to a subject induced to have hypoglycemia, and thereafter increasing the blood glucose level (patent document 2). However, a technique for delivering a drug more selectively to a desired site in the brain, for example, vascular endothelial cell or particular tissues or cells in the brain parenchyma has been desired.

It has been reported that the uptake of targeted nanoparticles in the brain increased by using a nanoparticle having a bond cleavable with an acid between transferrin and nanoparticle core (non-patent document 1). However, selective delivery of nanoparticles to particular tissues and cells in the brain is not described.

### [Document List]

### [Patent documents]

patent document 1: WO 2007/068429
patent document 2: WO 2015/075942

### [non-patent document]

non-patent document 1: Proc Natl Acad Sci U S A. 2015 Oct 6; 112 (40) :12486-91

### [SUMMARY OF THE INVENTION]

The present invention provides a drug delivery carrier and a composition containing same.

The present inventors have found that a drug can be more selectively delivered to a desired site in the brain, for example, vascular endothelial cells, and particular tissues and cells in the brain parenchyma by using a carrier containing a first block copolymer modified with a glucose transporter 1 (GLUT1) ligand and a second block copolymer modified with another ligand. The present invention is an invention based on this finding.

That is, according to the present invention, the following invention is provided.
[1] A carrier comprising a first block copolymer and a second block copolymer, wherein
   the first block copolymer is a block copolymer of a first non-charged segment and a first complex-forming segment, wherein at least some proportion of the first block copolymer is modified by a first molecule, and
   the second block copolymer is a block copolymer of a second non-charged segment and a second complex-forming segment, wherein at least some proportion of the second block copolymer is modified by a second molecule, and
   wherein the first molecule is a GLUT1 ligand, and the second molecule is different from the first molecule.
[2] The carrier of [1], wherein the first complex-forming segment and the second complex-forming segment are charged segments.
[3] The carrier of [2], wherein the second charged segment is charged oppositely to the electric charge of the first charged segment.
[4] The carrier of [2] or [3], wherein at least one of the first charged segment and the second charged segment is polyamino acid.
[5] The carrier of any of [1] - [4], wherein the chain length of the first non-charged segment is longer than the chain length of the second non-charged segment.
[6] The carrier of any of [1] - [4], wherein the chain length of the first non-charged segment is substantially the same as the chain length of the second non-charged segment.
[7] The carrier of any of [1] - [6], wherein the first block copolymer has a bond cleavable at pH 6.5 or less between the first non-charged segment and the first complex-forming segment.
[8] The carrier of any of [1] - [6], wherein the first block copolymer has a bond cleavable under a reductive environment between the first non-charged segment and the first complex-forming segment.
[9] The carrier of [8], wherein the bond cleavable under the reductive environment is a disulfide bond.
[10] The carrier of any of [1] - [9], wherein at least one of the first non-charged segment and the second non-charged segment is a polyalkylene glycol segment.
[11] The carrier of any of [1] - [10], wherein an orientation of the first non-charged segment and an orientation of the second non-charged segment are the same.
[12] The carrier of any of [1] - [11], wherein the first block copolymer envelops the second block copolymer.
[13] The carrier of any of [1] - [12], wherein the second molecule promotes uptake into a tissue or cell in the brain parenchyma.
[14] The carrier of [13], wherein the second molecule has specific affinity to a particular tissue or cell in the brain parenchyma.
[15] The carrier of any of [1] - [14], wherein the second molecule is aspartic acid or a derivative thereof.
[16] The carrier of any of [1] - [15], wherein the GLUT1 ligand is glucose.
[17] The carrier of [16], wherein the glucose is connected to the first non-charged segment via a carbon at position-6.
[18] The carrier of any of [1] - [17], which is a vesicle.
[19] The carrier of [18], wherein the vesicle has a diameter of 400 nm or less.
[20] The carrier of any of [1] - [19], wherein the chain length of the first non-charged segment is longer than the chain length of the second non-charged segment, and 10 mol% or more and less than 40 mol% of the first block copolymer is modified by GLUT1 ligand.
[21] The carrier of any of [1] - [19], wherein the chain length of the first non-charged segment is substantially the same as the chain length of the second non-charged segment, and 10 mol% or more and less than 40 mol% of a total of the first block copolymer and the second block copolymer is modified by GLUT1 ligand.
[22] The carrier of any of [1] - [19], wherein the chain length of the first non-charged segment is longer than the chain length of the second non-charged segment, and 40 - 100 mol% of the first block copolymer is modified by GLUT1 ligand.
[23] The carrier of any of [1] - [19], wherein the chain length of the first non-charged segment is substantially the same as the chain length of the second non-charged segment, and 40 - 99 mol% of a total of the first block copolymer and the second block copolymer is modified by GLUT1 ligand.
[24] A composition comprising the carrier of any of [1] - [23] and a drug encapsulated in the carrier.
[25] The composition of [24], wherein the drug is at least one drug selected from a bioactive substance, an antibody, a nucleic acid, a biocompatible fluorescence dye and a contrast agent.
[26] The composition of [24] or [25], which is for delivering the drug into the brain.
[27] The composition of [26], which is for delivering the drug to a brain vascular endothelial cell and/or brain parenchyma.
[28] The composition of [26], which is for delivering the drug to a particular tissue or cell in the brain parenchyma.
[29] The composition of [24] or [25], which is for passing the drug through a blood-brain barrier.
[30] The composition of [24] or [25], which is for passing the drug through a blood-nerve barrier, a blood-retinal barrier or a blood-cerebrospinal fluid barrier.
[31] The composition of any of [24] to [30], which is used for a treatment.
[32] The composition of any of [24] to [30], which is used for diagnosis.
[33] A method of delivering the drug into the brain of a subject, comprising administering the composition of [24] or [25] to the subject.
[34] The method of [33], wherein the drug is delivered to a brain vascular endothelial cell and/or brain parenchyma.
[35] The method of [34], wherein the drug is delivered to a particular tissue or cell in the brain parenchyma.
[36] A method of passing a drug through a blood-brain barrier of a subject, comprising administering the composition of [24] or [25] to the subject.
[37] A method of passing a drug through a blood-nerve barrier, a blood-retinal barrier or a blood-cerebrospinal fluid barrier of a subject, comprising administering the composition of [24] or [25] to the subject.

[1'] A carrier comprising a first block copolymer and a second block copolymer, wherein
   the first block copolymer is a block copolymer of a first polyethylene glycol and a first charged segment, which copolymer is modified by a first molecule and has a disulfide bond between the first polyethylene glycol and the first charged segment,
   the second block copolymer is a block copolymer of a second polyethylene glycol and a second charged segment, which copolymer is modified by a second molecule,
   the first polyethylene glycol has a chain length longer than that of the second polyethylene glycol,
   the first molecule is different from the second molecule, and the second charged segment is charged oppositely to the electric charge of the first charged segment.
[2'] The carrier of [1'], wherein the first block copolymer envelopes the second block copolymer.
[3'] The carrier of [2'], wherein the first polyethylene glycol and the second polyethylene glycol have the same orientation.
[4'] The carrier of any of [1'] - [3'], wherein at least one of the first charged segment and the second charged segment is polyamino acid.
[5'] The carrier of any of [1'] - [4'], wherein the first molecule is glucose.
[6'] The carrier of any of [1'] - [5'], wherein the second molecule is at least one kind selected from the group consisting of a low-molecular-weight compound, a peptide, a protein and an antibody.
[7'] A pharmaceutical composition comprising the carrier of any of [1'] - [6'] and a bioactive substance.
[8'] The pharmaceutical composition of [7'], which is for treating a brain disease or diagnosing a brain disease.

### [Effect of the Invention]

According to the present invention, a drug can be delivered more selectively to a desired site in the brain, for example, vascular endothelial cell or particular tissue or cell in the brain parenchyma.

### [Brief Description of the Drawings]

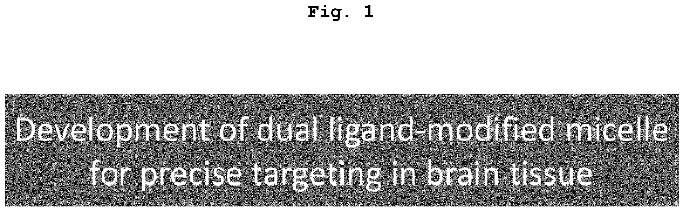

Fig. 1 shows the summary of the present invention.

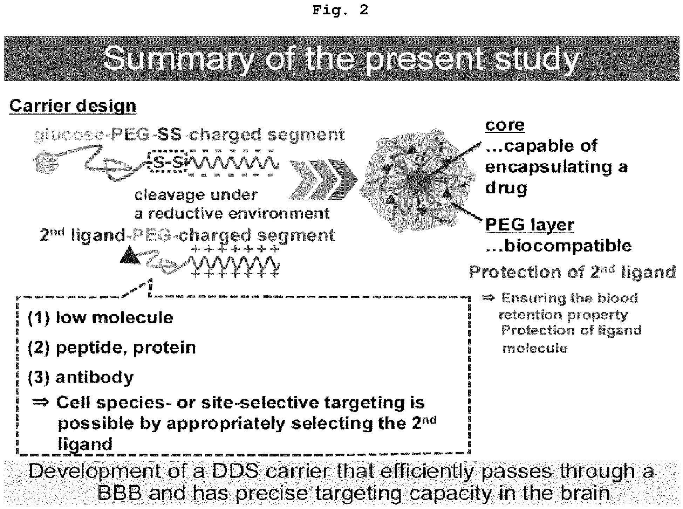

Fig. 2 shows the summary of the development of a DDS carrier that efficiently passes through a blood-brain barrier (BBB) and has precise targeting capacity in the brain. In previous researches, it was clarified that passing through BBB at high efficiency is possible by a glucose-modified micelle and control of the blood glucose level. However, the glucose-modified micelle is distributed throughout the brain after passing through BBB and does not show selectivity to a type of cell or site. Therefore, a novel carrier having precise targeting capacity in the brain tissue after passing through BBB was developed by appropriately selecting a second ligand in addition to the glucose ligand for passing through BBB. In this case, the second ligand can be protected in the blood as well as a decrease in the blood retention property by the second ligand and a decrease in the efficiency of passing through BBB can be prevented by setting the length of the polyethylene glycol (PEG) of the polymer on the side where glucose is conjugated longer than that of PEG of the polymer to which the second ligand is conjugated.

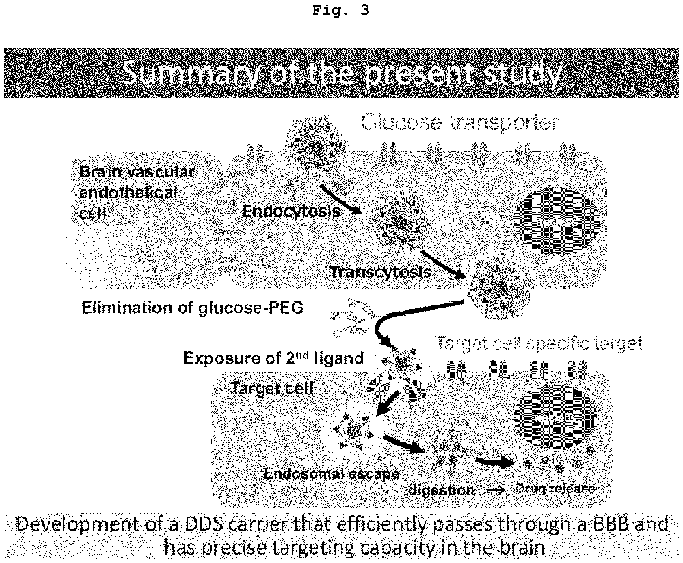

Fig. 3 shows one embodiment of efficient passage of the DDS carrier through the brain-blood vessel barrier (BBB) and precise targeting in the brain. Utilizing the fact that, after passing through BBB, glutathione having reducing activity is present in the brain parenchyma in an amount some tens to some hundreds of times higher than that in the blood, a system was conceived of in which a disulfide bond cleavable under a reducing environment is introduced between glucose-PEG and a charged segment (either positive or negative) so that glucose-PEG is eliminated after passing through BBB. This leads to the appearance of the second ligand alone after passing through BBB to allow for realization of higher cell type- or moiety-selectivity.

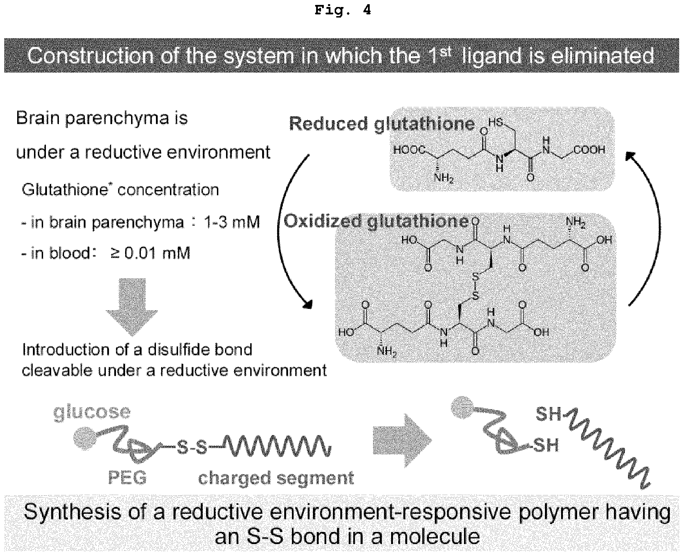

Fig. 4 shows synthesis of a reductive environment-responsive polymer having an S-S bond in a molecule. Utilizing the fact that, after passing through BBB, glutathione having a reducing action is present in the brain parenchyma in an amount some tens to some hundreds of times higher than that in the blood, a system was conceived of in which a disulfide bond cleavable under a reducing environment is introduced between glucose-PEG and a charged segment (either positive or negative) so that glucose-PEG is eliminated after passing through BBB. This leads to the appearance of the second ligand alone after passing through BBB to allow for realization of higher cell type- or moiety-selectivity.

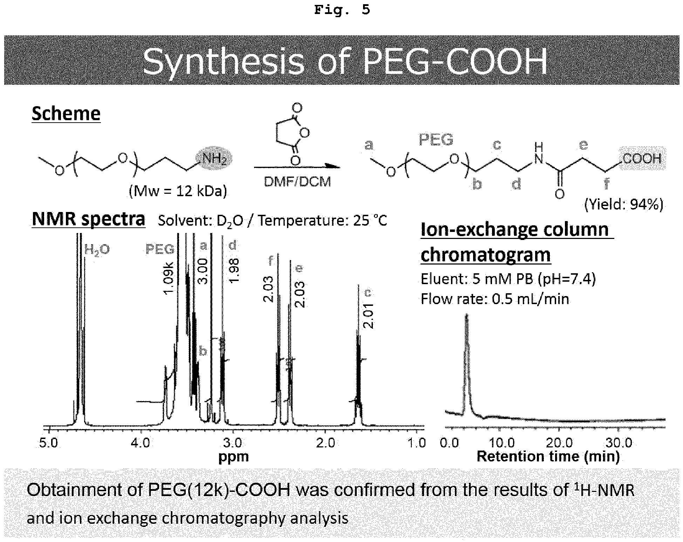

Fig. 5 shows a synthesis scheme of PEG-COOH. First, PEG-NH₂ (molecular weight of PEG 12 kDa, 800 mg, 0.0667 mmol, NOF CORPORATION) was dissolved in dichloromethane (16 mL, Nakarai Tesque, Inc.), succinic anhydride (140 mg, 0.714 mmol, Tokyo Chemical Industry Co., Ltd.) dissolved in DMF (4 mL, Nakarai Tesque, Inc.) was added, and the mixture was reacted at 35°C for - hours. The reaction solution was subjected to a dialysis treatment using a dialysis membrane having a fraction molecular weight of 6-8 kDa (Spectra/Por, Funakoshi Co., Ltd.) and lyophilized to give a white powder (yield 94%). This was analyzed by 1H-NMR (JEOL Ltd.) and ion exchange column chromatography. As a result, the reaction proceeded quantitatively and all terminal amino groups were confirmed to have been converted to carboxyl groups (PEG-COOH).

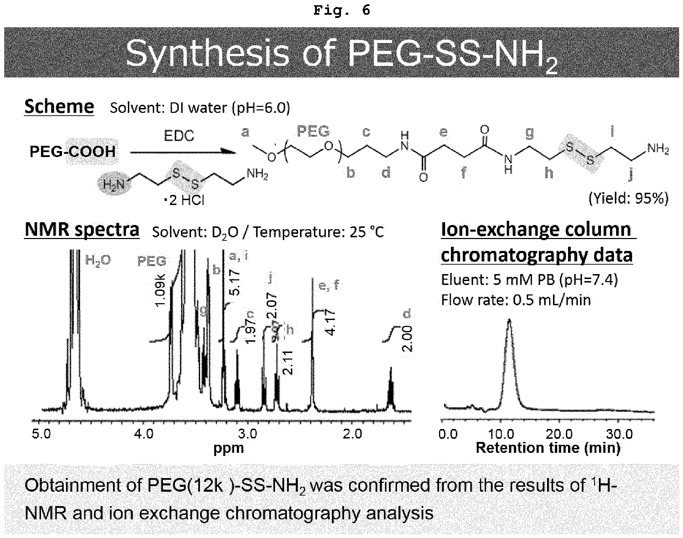

Fig. 6 shows a synthesis scheme of PEG-SS-NH₂. Next, PEG-COOH (500 mg, 0.0407 mmol) was dissolved in pure water (10 mL), the pH was adjusted to around neutral (6.5 - 7.5), cistamine dihydrochloride (183 mg, 0.976 mmol, Tokyo Chemical Industry Co., Ltd.) and 1-ethyl-3-(3-dimethylaminopropyl)carbodiimide (EDC) hydrochloride (156 mg, 1.05 mmol, Tokyo Chemical Industry Co., Ltd.) were added as powders, and the mixture was reacted at room temperature for 12 hr. The reaction solution was subjected to a dialysis treatment using a dialysis membrane having a fraction molecular weight of 6-8 kDa (Spectra/Por, Funakoshi Co., Ltd.) (outer liquid 0.01% HCl aq) and lyophilized to give a white powder (yield 94%). This was analyzed by 1H-NMR (JEOL Ltd.) and ion exchange column chromatography. As a result, the reaction proceeded quantitatively and it was found that a polymerization initiator (PEG-SS-NH_{2•}HCl) having a disulfide bond in a molecule as mentioned above was obtained.

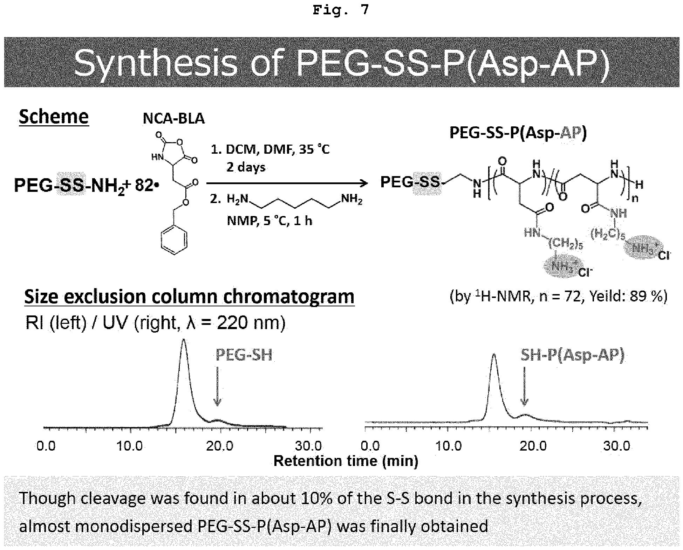

Fig. 7 shows a synthesis scheme of PEG-SS-P(Asp-AP). PEG-SS-NH2_{•}HCl was dissolved again in pure water, subjected to a dialysis treatment using a dialysis membrane having a fraction molecular weight of 6-8 kDa (outer liquid 0.01% aqueous ammonia solution), and lyophilized to give PEG-SS-NH2. Using this as an initiator, NCA-BLA was polymerized according to a previous report. To be specific, PEG-SS-NH2 (94 mg, 0.076 mmol) was dissolved in dichloromethane (0.7 mL, Kanto Chemical Co., Inc.), NCA-BLA (152 mg, 0.600 mmol) dissolved in a mixed solvent of DMF (0.3 mL, Kanto Chemical Co., Inc.) and dichloromethane (2 mL, Kanto Chemical Co., Inc.) was added, and the mixture was reacted at 35°C for 2 days. This reaction solution was added dropwise to hexane:ethyl acetate=2:3 (volume ratio, both from Godo Co., Ltd.), and the precipitate was recovered by suction filtration. This was dried under reduced pressure overnight to give PEG-SS-PBLA. Then, PEG-SS-PBLA (200 mg, 0.0069 mmol) was dissolved in NMP (10 mL, Nakarai Tesque, Inc.), and the solution was cooled to 5°C. Separately, 1,5-diaminopentane (6 mL, Tokyo Chemical Industry Co., Ltd.) was also dissolved similarly in NMP (6 mL, Nakarai Tesque, Inc.), the solution was cooled to 5°C, a polymer solution was added dropwise thereto, and the mixture was reacted at 5°C for 1 hr. The reaction solution was subjected to a dialysis treatment using a dialysis membrane having a fraction molecular weight of 15 kDa (Spectra/Por, Funakoshi Co., Ltd.) (outer liquid 0.01% HCl aq) and lyophilized to give a white powder (yield 89%, average degree of polymerization 72). This was analyzed by 1H-NMR (JEOL Ltd.) and size-exclusion column chromatography. As a result, it was found that a novel polymer (PEG-SS-P(Asp-AP)) having a disulfide bond between PEG and the charged segment was obtained almost quantitatively though cleavage was found in about 10% of the S-S bond.

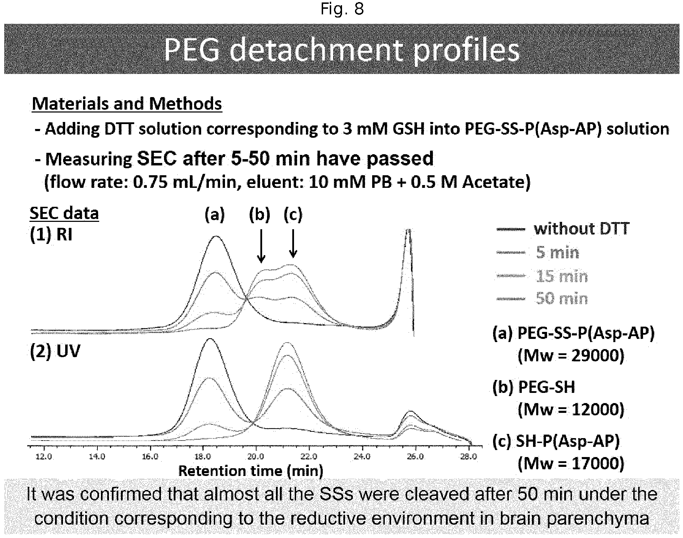

Fig. 8 shows evaluation of reductive environment responsiveness of PEG-SS-P(Asp-AP). To evaluate reductive environment responsiveness of the novel polymer (PEG-SS-P(Asp-AP)) synthesized earlier and having a disulfide bond between PEG and the charged segment, it was first dissolved in 10 mM phosphate buffer (pH=7.4) to 1 mg/mL, to which DTT solution was added to achieve a reductive environment of a level equivalent to the intracerebral glutathione concentration (3 mM), and the mixture was stirred by pipetting and left standing. This solution was sampled 5 min later, 15 min later and 50 min later, and changes in the molecular weight were evaluated by HPLC. As a result, the ratios of PEG-SH (20 min) and Sh-P(Asp-Ap) (22 min), which are fractions decomposed with the progress of time, increased and it was suggested that almost all S-S bonds were cleaved 50 min later.

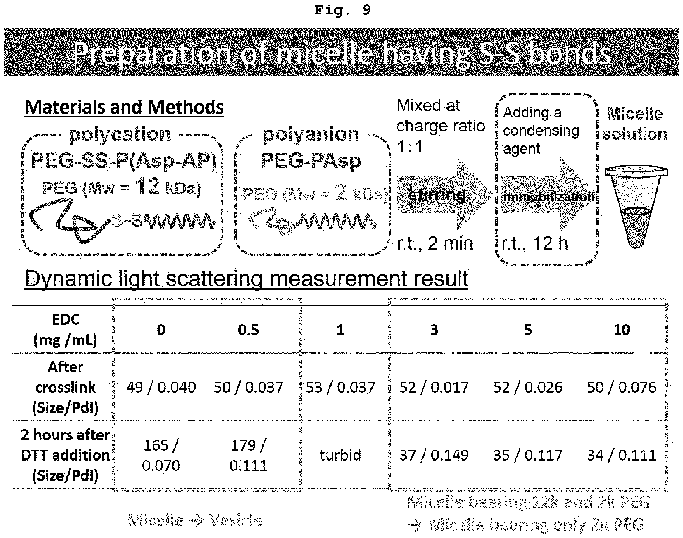

Fig. 9 shows a preparation scheme of a micelle having an S-S bond. A block copolymer (PEG-PAsp) composed of PEG (molecular weight 2Da) and an oppositely charged segment (negative charge chain this time), which is shorter than the novel polymer (PEG-SS-P(Asp-AP)) synthesized above and having a disulfide bond between PEG and the charged segment, was synthesized according to a previous report. These two kinds of polymers were each dissolved in 10 mM phosphate buffer to 1 mg/mL, mixed at a point of electric charge balance, and stirred at room temperature for 2 min, whereby a micelle covered with two kinds of PEGs having different lengths was prepared. Thereafter, a condensing agent EDC was dissolved in pure water to 1-10 mg/mL, and the same amount was added to the micelle solution to immobilize a core portion of the micelle (crosslinking). These micelles were each evaluated by dynamic light scattering measurement (Zetasizer, Malvern Instruments Ltd.) to find that monodispersed particles were obtained at an average particle size of about 50 nm. To these micelle solutions was added the same amount of a reducing agent dithiothreitol (DTT) (Wako Pure Chemical Industries, Ltd.) solution (5 mM). The mixture was stood at room temperature for 2 hr and a dynamic light scattering measurement was performed in the same manner. As a result, when the EDC concentration was 1 mg/mL or less, immobilization of the micelle core portion was not sufficient, and a change of form due to the elimination of PEG was confirmed. On the other hand, when a 3-10 mg/mL EDC solution was added, the core was sufficiently immobilized and a decrease in the particle size due to the elimination of PEG was confirmed while the shape of the micelle was maintained.

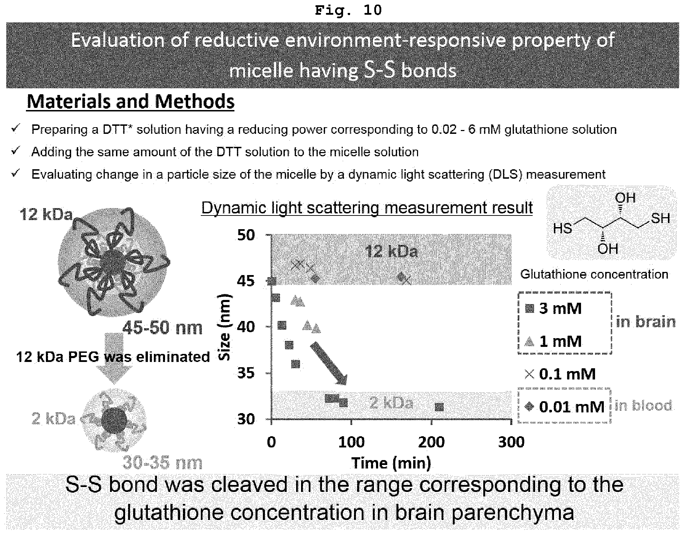

Fig. 10 shows evaluation of reductive environment responsiveness of a micelle having an S-S bond. Of the micelles prepared above, a micelle solution added with 10 mg/mL EDC whose core portion is expected to be strongly immobilized was further evaluated in detail for the reductive environment responsiveness. DTT was diluted with 10 mM phosphate buffer based on the reduction potential to give a DTT solution having a reducing power corresponding to a glutathione concentration of 0.02 - 6 mM. To the micelle solution was added the same amount of a DTT solution and a particle size change of the micelle was evaluated by a dynamic light scattering measurement. As a result, a decrease in the particle size was confirmed in the range (1-3 mM) corresponding to the glutathione concentration in the brain. This suggests that PEG having a molecular weight of 12 kDa was eliminated due to the cleavage of the disulfide bond and only PEG of 2 kDa remained on the surface layer. This hypothesis is supported by the fact that a micelle having 2 kDa PEG on the surface layer has a particle size of about 30 nm. Furthermore, the particle size did not change in the range (0.01 mM) corresponding to the glutathione concentration in the blood. Therefore, the micelle possibly maintains its structure stably in the blood and exhibits a function to eliminate PEG only in the brain parenchyma after passing through BBB.

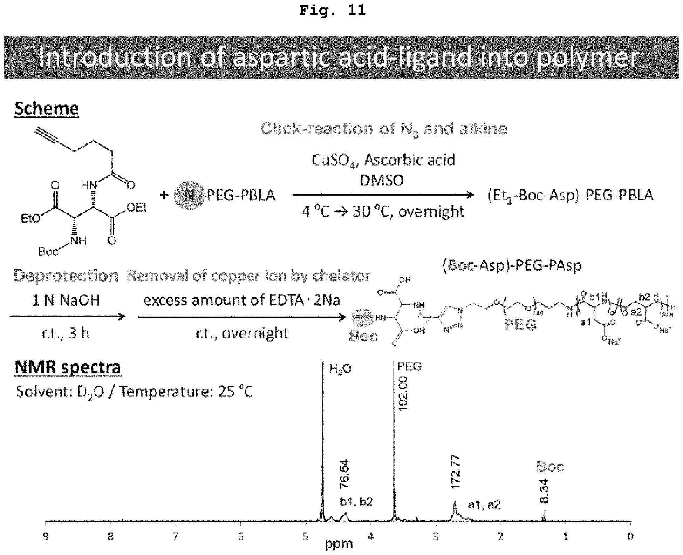

Fig. 11 shows a scheme of introduction of aspartic acid ligand into a polymer.

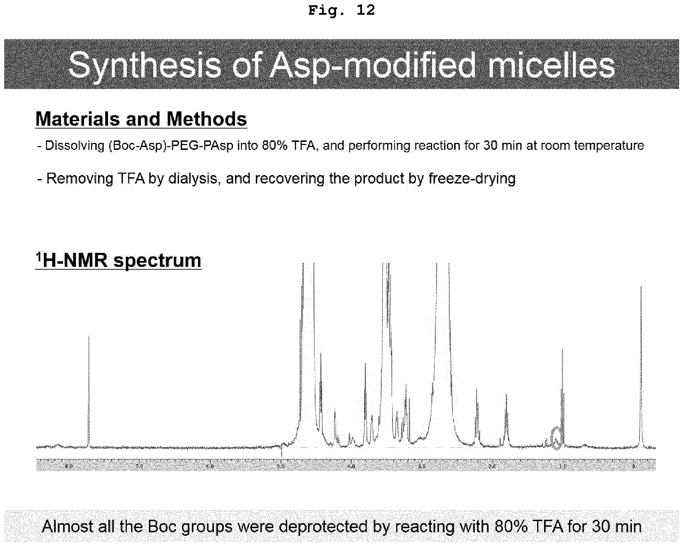

Fig. 12 shows elimination of Boc from (Boc-Asp)-PEG-PAsp.

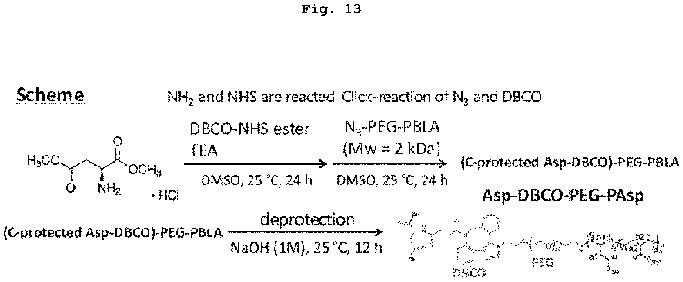

Fig. 13 shows a synthesis scheme of Asp-DBCO-PEG-PAsp.

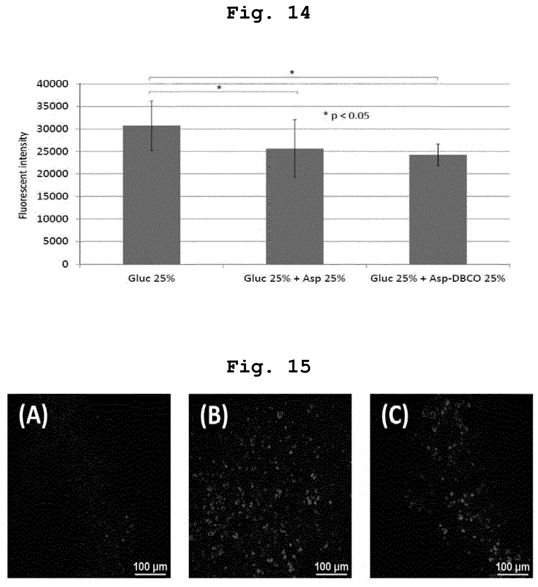

Fig. 14 shows uptake of micelle into MDA-MB-231 cells.
Fig. 15 shows distribution of micelles in brain sections. (A) Glc-modified micelle, (B) Asp-modified micelle, (C) Asp-DBCO-modified micelle.

### [Description of Embodiments]

### 1. Definition

In the present invention, the "drug transporter" means a carrier for drug delivery, which includes, for example, fine particles capable of encapsulating a drug such as vesicle, dendrimer, hydrogel, nanosphere and the like. Drug transporters generally have a size of 10 nm - 400 nm in diameter.

In the present specification, the "vesicle" intends micelle and hollow fine particle. The vesicle preferably has a biocompatible outer shell and an outer surface thereof is modified with a GLUT1 ligand, due to which the vesicle can interact with GLUT1.

In the present specification, the "micelle" means a vesicle formed by a monolayer molecular membrane. As the micelle, a micelle formed by an amphiphilic molecule such as surfactant and the like and a micelle formed by a polyion complex (PIC micelle) can be mentioned. It is known that an outer surface of micelle is preferably modified with polyethylene glycol from the viewpoint of blood retention time.

In the present specification, the "liposome" means a vesicle formed by bilayer of a molecular membrane. The molecular membrane is generally a bilayer membrane of phospholipid.

In the present specification, the "polyion complex polymersome" (hereinafter to be also referred to as "PICsome") means a fine particle which is formed by a polyion complex, has a void space enveloped by a membrane, and is capable of encapsulating a substance in the inside. It is known that an outer surface of PICsome is preferably modified with polyethylene glycol from the viewpoint of blood retention time.

In the present specification, the "polyion complex" (hereinafter to be also referred to as "PIC") means a complex formed by an anionic polymer (polyanion) and a cationic polymer (polycation), which are bonded or assembled by an electrostatic interaction. The complex may be poorly water-soluble or water-insoluble. For example, when a block copolymer of a non-charged polymer (e.g., PEG) and an anionic polymer, and a block copolymer of a non-charged polymer (e.g., PEG) and a cationic polymer are mixed in an aqueous solution to neutralize electric charge, a polyion complex is formed between the cationic segment and the anionic segment in the both block copolymers. The significance of linking the non-charged polymer such as PEG and the like to the above-mentioned charged polymer is suppression of the coagulation and precipitation of the polyion complex and resulting formation, by the polyion complex, of nano-fine particles having a particle size of some tens of nm and a monodispersed core-shell structure. In this case, non-charged segments such as PEG and the like cover the outer shell (shell) of the nano-fine particles, which is expected to conveniently increase biocompatibility and improve the blood retention time. In the polyion complex formation, moreover, one of the charged block copolymers does not require a non-charged segment (e.g., PEG portion) and may be replaced with homopolymer, surfactant, nucleic acid and/or enzyme. In the polyion complex formation, at least one of the anionic polymer and the cationic polymer forms a block copolymer with a non-charged polymer (e.g., PEG), and the both may form a block copolymer with a non-charged polymer (e.g., PEG). It is also known that a PIC micelle is easily formed when the PEG content is increased, and PICsome is easily formed when the PEG content is decreased. Examples of the anionic polymer (or segment) often used for the production of a polyion complex include polyglutamic acid, polyaspartic acid and nucleic acid (e.g., DNA, RNA (e.g., mRNA and siRNA) etc.), and examples of the cationic polymer (or segment) include polylysine and poly(5-aminopentyl aspartic acid). As used herein, mRNA means messenger RNA used for protein synthesis by translation, and siRNA means double stranded RNA (nucleic acid) capable of inducing RNA interference (RNAi). While siRNA is not particularly limited, it is a double stranded RNA consisting of 20 - 30 bp, preferably 21 - 23 bp, 25 bp, 27 bp, and having a sequence homologous with the target gene sequence.

In the present specification, the "block copolymer" means a polymer formed by plural polymers linked in tandem. Each polymer portion contained in the block copolymer is referred to as a segment or block. Each segment constituting the block copolymer may be linear or branched chain.

In the present specification, "for drug delivery" means being biocompatible and capable of encapsulating a drug in a vesicle. In the present specification, "for drug delivery" sometimes means a use utilizing an action to prolong the blood retention time of a drug as compared to the blood retention time of a naked drug.

In the present specification, "inducing hypoglycemia" means lowering the blood glucose level in a subject than that without the treatment. Examples of the method for inducing hypoglycemia include fasting, administration of antidiabetic drug, and the like. For example, when inducing hypoglycemia, for example, ingestion of other drugs, or drinks such as water and the like is acceptable as long as the object of induction of hypoglycemia is achieved. Induction of hypoglycemia may accompany other treatment substantially uninfluential on the blood glucose.

In the present specification, "fasting" means that the subject is fasted for, for example, not less than 3 hr, not less than 4 hr, not less than 5 hr, not less than 6 hr, not less than 7 hr, not less than 8 hr, not less than 9 hr, not less than 10 hr, not less than 11 hr, not less than 12 hr, not less than 13 hr, not less than 14 hr, not less than 15 hr, not less than 16 hr, not less than 17 hr, not less than 18 hr, not less than 19 hr, not less than 20 hr, not less than 21 hr, not less than 22 hr, not less than 23 hr, not less than 24 hr, not less than 25 hr, not less than 26 hr, not less than 27 hr, not less than 28 hr, not less than 29 hr, not less than 30 hr, not less than 31 hr, not less than 32 hr, not less than 33 hr, not less than 34 hr, not less than 35 hr, not less than 36 hr, not less than 37 hr, not less than 38 hr, not less than 39 hr, not less than 40 hr, not less than 41 hr, not less than 42 hr, not less than 43 hr, not less than 44 hr, not less than 45 hr, not less than 46 hr, not less than 47 hr or not less than 48 hr. Fasting causes hypoglycemia in the subject. The fasting period is determined by a doctor etc. by a doctor etc. in view of the health condition of the subject and, for example, a period not less than the time necessary for the subject to reach the fasting blood glucose is preferable. The fasting period may be set to a period not less than the time necessary for, for example, the expression of GLUT1 on the blood vessel inner surface of brain vascular endothelial cell to increase or reach plateau. The fasting period may be set to the above-mentioned period, for example, not less than 12 hr, not less than 24 hr or not less than 36 hr. In addition, fasting may accompany other treatment substantially uninfluential on the blood glucose level and expression of GLUT1 on the blood vessel inner surface.

In the present specification, "inducing an increase in the blood glucose level" means increasing the blood glucose level in a subject induced to have hypoglycemia or maintaining the hypoglycemia condition. The blood glucose level can be increased by various methods well known to those of ordinary skill in the art. For example, it can be increased by administration of a substance that induces an increase in the blood glucose level, for example, administration of a monosaccharide inducing an increase in the blood glucose level such as glucose, fructose, galactose and the like, administration of a polysaccharide inducing an increase in the blood glucose level such as maltose and the like, ingestion of carbohydrates inducing an increase in the blood glucose level such as starch and the like or by eating.

In the present specification, the "blood glucose operation" means inducing hypoglycemia in a subject and thereafter increasing the blood glucose level of the subject. After inducing hypoglycemia in a subject, the blood glucose level of the subject can be maintained in hypoglycemia. The time for maintaining the blood glucose level of the subject in hypoglycemia can be set to, for example, not less than 0 hr, not less than 1 hr, not less than 2 hr, not less than 3 hr, not less than 4 hr, not less than 5 hr, not less than 6 hr, not less than 7 hr, not less than 8 hr, not less than 9 hr, not less than 10 hr, not less than 11 hr, not less than 12 hr, not less than 13 hr, not less than 14 hr, not less than 15 hr, not less than 16 hr, not less than 17 hr, not less than 18 hr, not less than 19 hr, not less than 20 hr, not less than 21 hr, not less than 22 hr, not less than 23 hr, not less than 24 hr, not less than 25 hr, not less than 26 hr, not less than 27 hr, not less than 28 hr, not less than 29 hr, not less than 30 hr, not less than 31 hr, not less than 32 hr, not less than 33 hr, not less than 34 hr, not less than 35 hr, not less than 36 hr, not less than 37 hr, not less than 38 hr, not less than 39 hr, not less than 40 hr, not less than 41 hr, not less than 42 hr, not less than 43 hr, not less than 44 hr, not less than 45 hr, not less than 46 hr, not less than 47 hr or not less than 48 hr. Thereafter, the blood glucose level can be increased. In the present specification, when the "blood glucose is maintained", ingestion of other drugs, or drinks such as water and the like is acceptable as long as the object of maintenance of hypoglycemia in the subject is achieved. Induction of hypoglycemia may accompany other treatment substantially uninfluential on the blood glucose.

In the present specification, the "subject" may be a mammal inclusive of human. The subject may be a healthy subject or a subject affected with some disease. The disease here includes, for example, a motor neuron disorder, for example, Parkinson's disease and amyotrophic lateral sclerosis, and a cranial nerve disease, for example, psychotic disturbance, depression, mood disorder, anxiety, sleep disorder, dementia and substance-related disorder. While dementia is not particularly limited, Alzheimer's disease and Creutzfeldt-Jakob disease can be mentioned.

In the present specification, the "blood-brain barrier" refers to a functional barrier present between blood circulation and the brain and having selectivity to the permeation of substances. The entity of the blood-brain barrier is considered to be brain vascular endothelial cells and the like. There are many unclear points about the substance permeability of the blood-brain barrier. It is known that glucose, alcohol and oxygen easily pass through the blood-brain barrier, and it is considered that liposoluble substances and small molecules (for example, molecular weight less than 500) tend to more easily pass than water-soluble molecules and macromolecules (e.g., molecular weight not less than 500). Many therapeutic drugs for brain diseases and brain diagnostic drugs do not pass through the blood-brain barrier, which forms a major obstacle for the treatment of brain diseases, brain analysis and the like. In the present specification, the "blood-nerve barrier" refers to a functional barrier present between blood circulation and peripheral nerve and having selectivity to the permeation of substances. In the present specification, the "blood-cerebrospinal fluid barrier" refers to a functional barrier present between blood circulation and cerebrospinal fluid and having selectivity to the permeation of substances. In the present specification, the "blood-retinal barrier" refers to a functional barrier present between blood circulation and retinal tissue and having selectivity to the permeation of substances. The entity of the blood-nerve barrier, blood-cerebrospinal fluid barrier and blood-retinal barrier is considered to be vascular endothelial cells and the like present in the barrier, and the function thereof is considered to be the same as that of the blood-brain barrier.

In the present specification, the "GLUT1 ligand" means a substance that specifically binds to GLUT1. As a GLUT1 ligand, various ligands are known and is not particularly limited. For example, molecules such as glucose, hexose and the like can be mentioned. GLUT1 ligand can be used instead of glucose for the preparation of carrier or conjugate in the present invention. GLUT1 ligand preferably has affinity for GLUT1 which is equivalent to or higher than that for glucose. 2-N-4-(1-azi-2,2,2-trifluoroethyl)benzoyl-1,3-bis(D-mannose-4-yloxy)-2-propylamine (ATB-BMPA), 6-(N-(7-nitrobenz-2-oxa-1,3-diazol-4-yl)amino)-2-deoxyglucose (6-NBDG), 4,6-O-ethylidene-α-D-glucose, 2-deoxy-D-glucose, 3-O-methylglucose, 1,2-O-isopropylidene-α-D-glucofuranose are also known to bind to GLUT1, and these molecules (sometimes to be referred to as glucose derivatives) can also be used as GLUT1 ligand in the present invention.

In the present specification, the role of the GLUT1 ligand (glucose etc.) is considered to bind to GLUT1 which is a glucose transporter expressed on the blood vessel inner surface of vascular endothelial cells in the brain. Therefore, GLUT1 ligand other than glucose can also play the same role as that of glucose in the present invention. In the present invention, GLUT1 ligand can be placed on the carrier to appear on the outer surface so that it can bind to the glucose transporter expressed on the blood vessel inner surface of the vascular endothelial cells in the brain. Carriers (complex and vesicle and others) that can present GLUT1 ligand to GLUT1 are considered to be able to bind to GLUT1 and taken into the vascular endothelial cells together when GLUT1 is taken up into the cells by glucose after binding. When incorporated into vascular endothelial cells, the incorporated vesicles pass through the blood-brain barrier and migrate to the brain parenchyma. When glucose molecules that modify carriers were many, the proportion of vesicles that reached the brain parenchyma decreased slightly. This suggests that when glucose molecules that modify carriers are many, the carriers are incorporated into cells by endocytosis and directly pass through the cells toward the brain parenchyma, and that the efficiency of dissociation between the carriers and vascular endothelial cells decreases. In other words, a part of the carriers incorporated into cells by endocytosis are not dissociated from the brain vascular endothelial cells but accumulated in the brain vascular endothelial cells. Therefore, the carrier or composition of the present invention can also be used for delivery to the brain vascular endothelial cells. In addition, the role of the GLUT1 ligand in the present invention remains same in the blood-nerve barrier, blood-retinal barrier and blood-cerebrospinal fluid barrier. Particularly, in the blood-nerve barrier, blood-retinal barrier and blood-cerebrospinal fluid barrier, GLUT1 is expressed in vascular endothelial cells in hypoglycemia. Therefore, the carrier or composition of the present invention can be used for passing through the blood-nerve barrier, blood-retinal barrier and blood-cerebrospinal fluid barrier. The carrier or composition of the present invention can also be used for delivery to the vascular endothelial cells present in the blood-nerve barrier, blood-retinal barrier and blood-cerebrospinal fluid barrier.

### 2. Carrier

The present invention provides a carrier comprising a first block copolymer and a second block copolymer, wherein the first block copolymer is a block copolymer of a first non-charged segment and a first complex-forming segment, wherein at least some proportion of the first block copolymer is modified by a first molecule, and the second block copolymer is a block copolymer of a second non-charged segment and a second complex-forming segment, wherein at least some proportion of the second block copolymer is modified by a second molecule, and wherein the first molecule is a GLUT1 ligand, and the second molecule is different from the first molecule.

The present inventors have found that a drug can be selectively delivered to a desired site in the brain, for example, vascular endothelial cells, and particular tissues or cells in the brain parenchyma by using the above-mentioned carrier containing a first block copolymer modified with a GLUT1 ligand as a first molecule and, a second block copolymer modified with a second ligand as a second molecule, and reached the present invention.

The present inventors have already reported in detail in WO 2015/075942 (this specification as a whole is incorporated in full in the present specification) that a carrier having an outer surface modified with GLUT1 ligand such as glucose and the like causes accumulation in the brain. However, in the case of the carrier using such GLUT1 ligand alone as a targeting ligand, after passing through the blood-brain barrier, the carrier is generally transported to the neuron and/or microglia but the transport is not selective and the carrier is not transported to astrocyte. Furthermore, certain carriers are not incorporated into any cells. On the other hand, in the present invention, the GLUT1 ligand modifying the first block copolymer makes the carrier pass through the blood-brain barrier by the same mechanism as above, as well as the second ligand modifying the second block copolymer makes the carrier selectively transported to a desired site in the brain, for example, vascular endothelial cells, or particular tissues or cells in the brain parenchyma. Using such two kinds of ligands, entry into the brain and selective transport to a desired site in the brain are concurrently achieved. This is a surprising finding conventionally completely unknown and first obtained by the present inventors.

Specific explanations are given below.

The first block copolymer is a block copolymer of a first non-charged segment and a first complex-forming segment, and the second block copolymer is a block copolymer of a second non-charged segment and a second complex-forming segment. In the following, the first block copolymer and the second block copolymer are sometimes generically referred to simply as a block copolymer, the first non-charged segment and the second non-charged segment are sometimes generically referred to simply as a non-charged segment, and the first complex-forming segment and the second complex-forming segment are sometimes generically referred to simply as a complex-forming segment.

The non-charged segment is a polymer segment having non-charged properties. The "non-charged" here means that the segment is neutral as a whole. As an example, segment being free of positive or negative electric charge can be mentioned. Even when the segment has positive or negative charge in a molecule, if the topical effective charge density is not high and the electric charge of the whole segment is neutralized to the extent that formation of vesicles by self-organization is not prevented, it also corresponds to "non-charged".

The non-charged segment is preferably hydrophilic. As used herein, "hydrophilic" means being soluble to an aqueous medium.

The kind of the non-charged segment is not limited. It may be a segment consisting of single repeat units or a segment containing two or more kinds of repeat units at any combination and ratio. Specific examples of the non-charged segment include polyalkylene glycol, poly(2-oxazoline), polysaccharide, polyvinyl alcohol, polyvinylpyrrolidone, polyacrylamide, polymethacrylamide, polyacrylate, polymethacrylate, poly(2-methacryloxyethylphosphoryl choline), peptide, protein and derivatives thereof having an isoelectric point of around 7, and the like.

Among these, at least one, preferably both, of the first and the second non-charged segment is/are preferably polyalkylene glycol. Examples of the polyalkylene glycol include polyethylene glycol, polypropylene glycol and the like, with preference given to polyethylene glycol. Polyalkylene glycol may be linear or branched chain (e.g., PEGasus), preferably linear.

While the length of the non-charged segment is not particularly limited as long as selective delivery to a desired tissue or cell in the brain is possible, in the case of polyethylene glycol, it is a length corresponding to the degree of polymerization of generally not less than 5 (e.g., not less than 10, not less than 40) and not more than 20,000 (e.g., not more than 5,000, not more than 1,000, not more than 500, not more than 200). The length of the non-charged segment other than polyethylene glycol may be a length corresponding to polyethylene glycol having the aforementioned degree of polymerization.

The first non-charged segment and the second non-charged segment may be of the same kind or different kinds. In addition, the first non-charged segment and the second non-charged segment may each be a single kind of non-charged segment or a mixture of plural kinds of non-charged segments.

The complex-forming segment means a segment that interacts (bind etc.) with a component constituting the carrier, when the first block copolymer and the second block copolymer gather and form the carrier of the present invention, and contributes to the formation of the core structure of vesicles (micelle, liposome, PIC micelle, PICsome, polymersome, etc.). The complex-forming segment includes a hydrophobic segment and a charged segment. The hydrophobic segment contributes to the formation of carrier by forming micelle, liposome, polymersome and the like together with other hydrophobic segments via a hydrophobic interaction. The charged segment contributes to the formation of carrier by forming PIC micelle, PICsome and the like by polyion complex formation. The hydrophobic segments in the first and the second block copolymers are respectively referred to as the first hydrophobic segment and the second hydrophobic segment. The charged segments in the first and the second block copolymers are respectively referred to as the first charged segment and the second charged segment.

The kind of the hydrophobic segment is not limited. It may be a segment consisting of single repeat units or a segment containing two or more kinds of repeat units at any combination and ratio. Examples of the hydrophobic segment include, but are not limited to, polylactic acid, polyamino acid containing hydrophobic amino acid having a hydrophobic group in the side chain as a constitution unit or a derivative thereof and the like. As the hydrophobic amino acid having a hydrophobic group in the side chain, amino acid showing solubility in water (100 g) at 25°C of preferably not more than 5 g, further preferably not more than 4 g, can be mentioned. Examples of such amino acid include non-polar natural amino acids such as leucine, isoleucine, phenylalanine, methionine, tryptophan and the like, and hydrophobic derivatives of amino acid introduced with a hydrophobic group in the side chain. As the hydrophobic derivative of amino acid, hydrophobic derivatives of acidic amino acids such as aspartic acid, glutamic acid and the like can be preferably mentioned. As the above-mentioned hydrophobic group to be introduced, a saturated or unsaturated liner or branched aliphatic hydrocarbon group having a carbon number of 6 - 27, and an aromatic hydrocarbon group having a carbon number of 6 - 27 or steryl group are preferably recited as examples. Examples of the polyamino acid containing hydrophobic amino acid having a hydrophobic group in the side chain as a constitution unit include poly(benzyl-L-aspartate) (PBLA) .

On the other hand, the charged segment is a segment having an electric charge. It is divided into a cationic segment and an anionic segment depending on the kind of the electric charge of the segment as a whole.

The cationic segment is a polymer segment having a cation group and cationic property (cationicity). It may optionally have an anion group somewhat as long as the cationic property of the cationic segment as a whole is not interfered.

The kind of the cationic segment is not limited. It may be a segment consisting of single repeat units or a segment containing two or more kinds of repeat units at any combination and ratio. As the cationic segment, a polymer comprising a monomer unit containing an amino group and the like can be mentioned, but it is not particularly limited thereto. The monomer unit containing an amino group is preferably a monomer unit containing an amino group in the side chain. Examples of the polymer comprising a monomer unit containing an amino group include, but are not particularly limited to, polyamine, polyamino acid comprising amino acid containing an amino group in the side chain as a monomer unit or a derivative thereof and the like, with preference given to polyamino acid comprising amino acid containing an amino group in the side chain as a monomer unit or a derivative thereof. Examples of the polyamino acid comprising amino acid containing an amino group in the side chain as a monomer unit or a derivative thereof include, but are not particularly limited to, polyaspartamide, polyglutamide, polylysine, polyarginine, polyhistidine, and derivatives thereof and the like, with preference given to polyaspartamide or a derivative thereof and polyglutamide or a derivative thereof. Poly(Asp-AP) (or poly(Glu-AP) obtained by introducing aminopentane (AP) into the carboxylic acid in the side chain of aspartic acid (or glutamic acid) by reacting polyaspartic acid (or polyglutamic acid) with 1,5-diaminopentane; poly(Asp-DET)(or poly(Glu-DET)) obtained by introducing DET into the carboxylic acid in the side chain of aspartic acid (or glutamic acid) by reacting polyaspartic acid (or polyglutamic acid) with DET (H₂NCH₂CH₂NH-CH₂CH₂NH₂) and the like are preferably used.

On the other hand, the anionic segment is a polymer segment having an anion group and anionic property (anionicity). It may optionally have a cation group somewhat as long as the anionic property of the anionic segment as a whole is not interfered.

The kind of the anionic segment is not limited. It may be a segment consisting of single repeat units or a segment containing two or more kinds of repeat units at any combination and ratio. Examples of the anionic segment include, but are not limited to, a polymer comprising a monomer unit containing a carboxyl group, a polymer comprising a monomer unit containing a sulfate group, a polymer comprising a monomer unit containing a phosphate group and the like. The monomer unit containing a carboxyl group is preferably a monomer unit containing a carboxyl group in the side chain. The monomer unit containing a sulfate group is preferably a monomer unit containing a sulfate group in the side chain. Examples of the polymer comprising a monomer unit containing a carboxyl group in the side chain include, but are not limited to, polyamino acid containing amino acid having a carboxyl group in the side chain as a monomer unit or a derivative thereof and the like. Examples of the polymer comprising a monomer unit containing a sulfate group in the side chain include, but are not limited to, sulfated polysaccharides and the like. Examples of the polymer comprising a monomer unit containing a phosphate group in the side chain include, but are not limited to, nucleic acid and the like. The anionic segment is preferably a polyamino acid containing amino acid having a carboxyl group in the side chain as a monomer unit or a derivative thereof, nucleic acid or the like.

Examples of the polyamino acid containing amino acid having a carboxyl group in the side chain as a monomer unit or a derivative thereof include, but are not limited to, polyaspartic acid, polyglutamic acid, polyamino acid obtained by reacting an adequate amount of aconitic acid anhydride or citraconic anhydride with the side chain amino group of the aforementioned polyamino acid containing amino acid having an amino group in the side chain as a monomer unit or a derivative thereof, and derivatives thereof and the like. The polyamino acid containing amino acid having a carboxyl group in the side chain as a monomer unit or a derivative thereof is preferably polyaspartic acid or polyglutamic acid.

As the nucleic acid, a single strand or double stranded DNA or RNA can be mentioned. The nucleic acid may be a functional nucleic acid suitable for the use of the vesicle. As the functional nucleic acid, siRNA, miRNA (microRNA), antisense RNA, antisense DNA, ribozyme, DNA enzyme and the like can be mentioned. These are selected according to the use of the vesicle. For example, when the vesicle is used for DDS of RNAi, siRNA is used as a nucleic acid. The nucleic acid may be modified.

While the length of the complex-forming segment is not particularly limited as long as selective delivery to a desired tissue or cell in the brain is possible, in the case of polyamino acid, the degree of polymerization is generally not less than 5 (e.g., not less than 10, not less than 40) and not more than 20,000 (e.g., not more than 5,000, not more than 1,000, not more than 500, not more than 200). The length of the complex-forming segment other than polyamino acid may be a length corresponding to the polyamino acid having the aforementioned degree of polymerization.

The first complex-forming segment and the second complex-forming segment may be of the same kind or different kinds. In addition, the first complex-forming segment and the second complex-forming segment may each be a single kind of complex-forming segment or a mixture of plural kinds of complex-forming segments.

When the complex-forming segment is a charged segment, the first charged segment and the second charged segment may be of the same kind or different kinds. In addition, the first charged segment and the second charged segment may each be a single kind of charged segment or a mixture of plural kinds of charged segments. In addition, the first charged segment and the second charged segment may have the same electric charge (i.e., both are cationic segments or both are anionic segments), or may have the opposite electric charge (i.e., one is a cationic segment and the other is an anionic segment). The first charged segment and the second charged segment preferably have the opposite electric charge (i.e., one is a cationic segment and the other is an anionic segment). In addition, at least one, preferably both, of the first charged segment and the second charged segment is/are more preferably polyamino acid or a derivative thereof.

In the present invention, at least some proportion of the first block copolymer is modified with a GLUT1 ligand (i.e., GLUT1 ligand is linked to at least some proportion of the first block copolymer). As the GLUT1 ligand, those mentioned above can be recited, with preference given to glucose. One molecule of GLUT1 ligand may be linked to one molecule of the first block copolymer, or plural molecules of GLUT1 ligand may be linked to one molecule of the first block copolymer. The GLUT1 ligand-linking site in the first block copolymer is not particularly limited as long as selective delivery of the carrier of the present invention into the brain is possible. To efficiently present GLUT1 ligand on the outer surface of the carrier, the first non-charged segment in the first block copolymer is preferably modified with GLUT1 ligand. While the site to be modified with GLUT1 ligand in the first non-charged segment is not particularly limited, to efficiently present GLUT1 ligand on the outer surface of the carrier, the GLUT1 ligand is linked to the terminal of the first non-charged segment (the side not linked with the first complex-forming segment). That is, in a preferable embodiment, in the first block copolymer, GLUT1 ligand, the first non-charged segment and the first complex-forming segment are linked in the order of GLUT1 ligand-the first non-charged segment-the first complex-forming segment from the terminal.

When glucose is used as the GLUT1 ligand, glucose can link to the first block copolymer (preferably, to the first non-charged segment, more preferably, to the terminal of the first non-charged segment) via any one of the carbon atoms at position 1, position 2, position 3, position 4, and position 6 thereof. It is known that OH groups, substituents at the carbon atom at position 1, position 3 and position 4, are deeply involved in the binding of GLUT1 and glucose. Therefore, it is preferable to link glucose to the first block copolymer (preferably, to the first non-charged segment, more preferably, to the terminal of the first non-charged segment) via a carbon atom at position 2 or position 6 having low involvement in the binding with GLUT1. By linking glucose to the first block copolymer via a carbon atom at position 2 or position 6 having low involvement in the binding with GLUT1, the uptake efficiency of the carrier into the brain is expected to be improved (WO 2015/075942). In the present specification, glucose conjugated via a carbon atom at position n is sometimes referred to as "Glc(n)" {n is an integer of any of 1 - 4 and 6}. For example, in the present specification, glucose conjugated via the carbon atom at position 6 is sometimes referred to as "Glc(6)", glucose conjugated via the carbon atom at position 2 is sometimes referred to as "Glc(2)", and glucose conjugated via the carbon atom at position 3 is sometimes referred to as "Glc(3)".

The first block copolymer may be modified in the entirety with the GLUT1 ligand or only some proportion thereof may be modified with the GLUT1 ligand. In the following, the first block copolymer modified with the GLUT1 ligand is abbreviated as the modified first block copolymer, and the first block copolymer not modified with the GLUT1 ligand is abbreviated as the unmodified first block copolymer. The proportion of the modified first block copolymer to the first block copolymer is generally 10 - 100%, and can be appropriately changed according to the site in the brain intended for delivery, coupling manner of non-charged segment and complex-forming segment and the like (described in detail below).

In the present invention, at least some proportion of the second block copolymer is modified with the second molecule (the second ligand) (i.e., the second molecule is linked to at least some proportion of the second block copolymer). The second molecule is a ligand different from the aforementioned first molecule (i.e., GLUT1 ligand). One molecule of the second molecule may be linked to 1 molecule of the second block copolymer, or plural molecules of the second molecule may be linked to 1 molecule of the second block copolymer. While the second molecule linking site in the second block copolymer is not particularly limited as long as selective delivery of the carrier of the present invention to a desired tissue or cell in the brain is possible, preferably, the second non-charged segment in the second block copolymer is modified with the second molecule to efficiently present the second molecule on the outer surface of the carrier after transported into the brain. While the site to be modified with the second molecule in the second non-charged segment is not particularly limited, the second molecule is linked to the terminal of the second non-charged segment (side to which the second complex-forming segment is not linked) to efficiently present the second molecule on the outer surface of the carrier after transported into the brain. That is, in a preferable embodiment, in the second block copolymer, the second molecule, the second non-charged segment and the second complex-forming segment are linked in the order of the second molecule-the second non-charged segment-the second complex forming segment from the terminal.

The second molecule modifying the second block copolymer, i.e., the second ligand, may be a compound (ligand) having a specific affinity for a particular molecule (e.g., receptor, transporter, cellular surface marker etc.) expressed on the surface of a desired tissue or cell in the brain (sometimes to be referred to as target tissue or target cell). As the second molecule, various ligands such as lower molecule, antibody, aptamer, peptide and the like can be used, and a ligand having an affinity specific to a molecule expressed on the surface of a desired tissue or cell in the brain can be appropriately selected depending on the intended tissue or cell into which a carrier is selectively transported. Specific examples of the desired tissue or cell in the brain include, but are not limited to, hippocampus and cerebellum as sites in the brain parenchyma, neuronal cells (neuron, astrocyte, microglia etc.) and vascular endothelial cell and the like as cells in the brain parenchyma. It is also possible to desire, for example, further species-selective transport among the neurons. Specific examples of a ligand having specific affinity for a molecule expressed on the surface of such desired tissue or cell in the brain include a ligand having specific affinity for a neurotransmitter receptor expressed on neuron and the like in the brain parenchyma, an antibody or a binding fragment thereof having specific affinity for a cellular surface marker of a particular cell in the brain parenchyma and the like. For example, using aspartic acid or a derivative thereof having specific affinity for glutamate receptor as the second molecule, selective delivery to a glutamate receptor-expressing cell becomes possible. Glutamate receptors are expressed in various cells in the brain parenchyma (e.g., neuronal cells (neuron, glial cell etc.), vascular endothelial cell), and uptake of carrier into these cells in the brain parenchyma is enhanced by using aspartic acid or a derivative thereof as the second molecule. Therefore, the problem of insufficient uptake of carrier into brain parenchymal cells due to floating carriers in the gaps in the brain parenchyma may be solved. The subtype and expression level of glutamate receptor vary depending on the cell kinds and site, and the recognizable structure is different for each subtype. Using a ligand for each subtype, targeting of the cell kind and site can be achieved. Receptors for low-molecular-weight (e.g., molecular weight of not more than 1000) neurotransmitters such as amino acid, acetylcholine, monoamine and the like are specifically recognized by low-molecular-weight ligands. Such a low-molecular-weight ligand can be easily handled during production of the second block copolymer and the like and influence on the structure and stability of the whole carrier can be suppressed due to the low molecular weight. Therefore, such ligand is preferable.

When aspartic acid or a derivative thereof is used as the second molecule, the residue of aspartic acid or a derivative thereof in the second block copolymer is a group represented by, for example, the following formula: 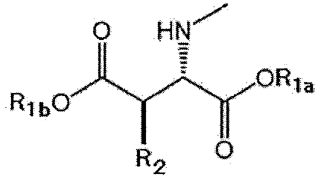 wherein R₁ₐ and R_{1b} are each independently a hydrogen atom or an unsubstituted or substituted liner or branched C₁₋₁₂ alkyl group (preferably, C₁₋₆ alkyl group), and R₂ is a hydrogen atom or an unprotected or protected amino group. Examples of the liner or branched C₁₋₁₂ include methyl, ethyl, n-propyl, iso-propyl, n-butyl, sec-butyl, tert-butyl, n-pentyl, n-hexyl, decyl, undecyl and the like. As the substituent when the alkyl group is substituted, acetalized formyl group, cyano group, formyl group, carboxyl group, amino group, C₁₋₆ alkoxycarbonyl group, C₂₋₇ acylamido group, the same or different tri-C₁₋₆ alkylsiloxy group, siloxy group or silylamino group can be mentioned. Acetalization means formation of an acetal moiety by a reaction between carbonyl of formyl and two molecules of alkanol having a carbon number of 1 - 6 or optionally branched alkylenediol having a carbon number of 2 - 6. It is also a protection method of the carbonyl group. For example, when the substituent is an acetalized formyl group, it can be hydrolyzed under mild acidic conditions and converted to a formyl group (-CHO) (or aldehyde group) which is another substituent. Examples of the protecting group when the amino group is protected include, but are not limited to, tert-butoxycarbonyl group (-Boc), benzyloxycarbonyl group (-Z or Cbz), 9-fluorenylmethyloxycarbonyl group (-Fmoc), 2,2,2-trichloroethoxycarbonyl group (-Troc), allyloxycarbonyl group (-Alloc), trifluoroacetyl group (CF₃CO-), phthaloyl group (-Pht), p-toluenesulfonyl group (-Ts or Tos), 2-nitrobenzenesulfonyl group (-Ns) and the like.

Preferably, R₁ₐ and R_{1b} are each independently a hydrogen atom or a C₁₋₁₂ alkyl group (preferably, C₁₋₆ alkyl group) and R₂ is a hydrogen atom or an unprotected or protected amino group. Examples of the protecting group when the amino group is protected include tert-butoxycarbonyl group (-Boc), benzyloxycarbonyl group (- Z or Cbz), 9-fluorenylmethyloxycarbonyl group (-Fmoc), 2,2,2-trichloroethoxycarbonyl group (-Troc), allyloxycarbonyl group (-Alloc), trifluoroacetyl group (CF₃CO-), phthaloyl group (-Pht), p-toluenesulfonyl group (-Ts or Tos), 2-nitrobenzenesulfonyl group (-Ns) and the like.

This aspartic acid or a derivative thereof does not have a particular modification at the recognition site by glutamate receptors. Thus, it can be recognized by various subtypes of glutamate receptors. Irrespective of the kind of subtype of the glutamate receptor expressed, therefore, it can enhance uptake of carrier by various cells in the brain parenchyma (e.g., neuronal cells (neuron, glial cell etc.), vascular endothelial cell).

In one embodiment, a K_{D} value with respect to the binding affinity of the second molecule to a molecule expressed on a surface of a desired tissue or cell in the brain is not more than 1×10⁻³ M (e.g., not more than 1×10⁻⁴ M, not more than 1×10⁻⁵ M, not more than 1×10⁻⁶ M, not more than 1×10⁻⁷ M, not more than 1×10⁻⁸ M, not more than 1×10⁻⁹ M, not more than 1×10⁻¹⁰ M, not more than 1×10⁻¹¹ M) .

The second block copolymer may be modified in the entirety with the second molecule or only some proportion thereof may be modified with the second molecule. In the following, the second block copolymer modified with the second molecule is abbreviated as the modified second block copolymer, and the second block copolymer not modified with the second molecule is abbreviated as the unmodified second block copolymer. The proportion of the modified second block copolymer to the second block copolymer is generally not less than 10%, preferably not less than 20%, not less than 30%, not less than 40%, not less than 50%, not less than 60%, not less than 70%, not less than 80%, not less than 90%, not less than 95%, most preferably 100%.

The first block copolymer may have (1) a stable bond uncleavable in blood, vascular endothelial cell and brain parenchyma, (2) a bond cleavable at pH 6.5 or below, or (3) a bond cleavable under a reductive environment, between the first non-charged segment and the first complex-forming segment. A stable bond hardly degraded in the body is well known to those of ordinary skill in the art, and this can be used as a stable bond uncleavable in blood, vascular endothelial cell and brain parenchyma. The first non-charged segment and the first complex-forming segment may be directly covalently bonded or bonded via a linker. A linker can provide a distance corresponding to, for example, 1 - 30, preferably 1 - 10 carbon-carbon bonds. A bond cleavable at pH 6.5 or below and a bond cleavable under a reductive environment are described in detail below.

In the second block copolymer, the bond between the second non-charged segment and the second complex-forming segment is preferably a stable bond uncleavable in blood, vascular endothelial cell and brain parenchyma. Preferably, a bond cleavable at pH 6.5 or below and a bond cleavable under a reductive environment are not present between the second non-charged segment and the second complex-forming segment. The second non-charged segment and the second charged segment may be directly covalently bonded or bonded via a linker. A linker can provide a distance corresponding to, for example, 1 - 30, preferably 1 - 10 carbon-carbon bonds.

The GLUT1 ligand modification in the first block copolymer is preferably via a stable bond uncleavable in blood, vascular endothelial cell and brain parenchyma. Preferably, a bond cleavable at pH 6.5 or below and a bond cleavable under a reductive environment are not present between the first block copolymer and the GLUT1 ligand. The first block copolymer and the GLUT1 ligand may be directly covalently bonded or bonded via a linker. A linker can provide a distance corresponding to, for example, 1 - 30, preferably 1 - 10 carbon-carbon bonds.

The modification with the second molecule in the second block copolymer is preferably via a stable bond uncleavable in blood, vascular endothelial cell and brain parenchyma. Preferably, a bond cleavable at pH 6.5 or below and a bond cleavable under a reductive environment are not present between the second block copolymer and the second molecule. The second block copolymer and the second molecule may be directly covalently bonded or bonded via a linker. A linker can provide a distance corresponding to, for example, 1 - 30, preferably 1 - 10 carbon-carbon bonds.

According to the carrier of the present invention, at least some proportion (e.g., not less than 5%, preferably not less than 10%, more preferably not less than 25%) of the GLUT1 ligand is preferably exposed on the outer surface of the carrier at least during passage through the blood-brain barrier so that the GLUT1 ligand can function. Also, at least some proportion (e.g., not less than 5%, preferably not less than 10%, more preferably not less than 25%) of the second ligand is preferably exposed on the outer surface of the carrier at least in a desired site in the brain into which the carrier is to be selectively transported after passage through the blood-brain barrier. While an embodiment achieving such exposed state of the ligand on the outer surface of the carrier is not particularly limited, the following two embodiments are preferably mentioned.

### (1) First embodiment:

In the first embodiment, the first and the second non-charged segments have substantially the same chain length. Substantially the same means that, for example, the difference in the chain length is not more than ±20%, preferably not more than ±10%. In this embodiment, the bond between the first non-charged segment and the first complex-forming segment may be a stable bond uncleavable in blood, vascular endothelial cell and brain parenchyma or a bond specifically cleavable under environmental conditions of a desired site in the brain where a carrier is to be selectively transported or a site to be passed through before reaching a desired site in the brain where a carrier is to be selectively transported (e.g., under environmental conditions in the brain parenchyma or endosome in vascular endothelial cell) (described in detail below). In this embodiment, GLUT1 ligand and the second ligand are simultaneously exposed on the outer surface of a carrier. As a result, the carrier binds to GLUT1 on brain vascular endothelial cell via a GLUT1 ligand, passes through the blood-brain barrier, moves into the brain parenchyma, and successively binds to the surface of a desired tissue or cell in the brain parenchyma via the second ligand on the carrier.

### (2) Second embodiment:

In the second embodiment, the first non-charged segment conjugated with a GLUT1 ligand is specifically cleaved under environment conditions of a desired site in the brain where the carrier is to be selectively transported or a site to be passed through before reaching a desired site in the brain where the carrier is to be selectively transported (e.g., under environment conditions in the brain parenchyma or endosome in vascular endothelial cell), thereby the second ligand is subsequently exposed on the outer surface of the carrier or the ratio of the exposure is increased. In this embodiment, in the aforementioned first block copolymer, the aforementioned first non-charged segment and the aforementioned first complex-forming segment are linked by a bond specifically cleavable under environmental conditions of a desired site in the brain where the carrier is to be selectively transported or a site to be passed through before reaching a desired site in the brain where a carrier is to be selectively transported (e.g., under environmental conditions in the brain parenchyma or endosome in vascular endothelial cell) (the first block copolymer having such bond is hereinafter to be abbreviated as the cleavable first block copolymer and the first block copolymer not having such bond is hereinafter to be abbreviated as the uncleavable first block copolymer). Preferable examples include a responsive functional group cleavable in the endosome of vascular endothelial cell and a responsive functional group cleavable in the brain parenchyma.

As the responsive functional group cleavable in the endosome of vascular endothelial cell, a bond cleavable due to a pH change can be mentioned. That is, the pH in the brain blood vessel is about 7.0 - 7.4 and that in the endosome is about 6.5 or below. A bond uncleavable under pH environment of about 7.0 - 7.4 and cleavable under pH environment of about 6.5 or less can be used. Examples include

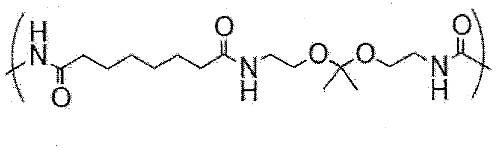

(Proc Natl Acad Sci U S A. 2015 Oct 6; 112(40):12486-91), -O-C(CH₃)₂-O- and the like. In this embodiment, the first non-charged segment conjugated with GLUT1 ligand is cleaved in the endosome of a vascular endothelial cell. As a result, GLUT1 ligand disappears from the outer surface of the carrier, the carrier is released from GLUT1 on the inner surface of the endosome, and can move freely. Consequently, release into the brain parenchyma by transcytosis is promoted. In addition, the above-mentioned cleavage leads to the subsequent exposure of the second ligand on the outer surface, the carrier binds to a desired tissue or cell in the brain parenchyma via the exposed second ligand.

As the bond cleavable in the brain parenchyma, a bond cleavable under a reductive environment can be mentioned. The "bond cleavable under a reductive environment" means a bond cleavable under a reductive environment equivalent to a reduced glutathione (GSH) concentration of not less than 0.02 mM, preferably not less than 0.1 mM, more preferably not less than 1 mM, further preferably not less than 3 mM. Whether a bond corresponds to "a bond cleavable under a reductive environment" can be evaluated by, for example, dissolving a compound having a bond to be evaluated in a DTT solution (in 10 mM phosphate buffer (pH=7.4)), which confers a reductive environment equivalent to a reduced glutathione (GSH) concentration of not less than 0.02 mM (e.g., 0.02 mM, 0.1 mM, 1 mM, 3 mM), incubating the solution for 50 min at room temperature (25°C), and comparing the residual amount of an undecomposed compound having the bond to be evaluated remaining uncleaved with that without DTT addition. In this evaluation system, when the residual amount of the undecomposed compound is, for example, not more than 30%, preferably not more than 20%, more preferably 10% or less, of that without addition of DTT, the evaluated bond is taken as "a bond cleavable under reductive environment". The inside of the brain blood vessel is a non-reductive environment having, for example, a glutathione concentration of about 0.01 mM, and the inside of the brain parenchyma is a reductive environment having, for example, a glutathione concentration of about 1 - 3 mM. A bond uncleavable under a non-reductive environment having a glutathione concentration of about 0.01 mM and cleavable under a reductive environment having a glutathione concentration of about 1 - 3 mM may be used. Examples thereof include, but are not limited to, a disulfide bond. In this embodiment, the carrier is delivered into the brain parenchyma due to transcytosis, and the first non-charged segment conjugated with GLUT1 ligand is cleaved in the brain parenchyma. As a result, GLUT1 ligand disappears from the outer surface of the carrier, the carrier is released from GLUT1 on the inner surface of the endosome, and can move freely, and release of the carrier into the brain parenchyma is promoted. In addition, the above-mentioned cleavage causes subsequent exposure of the second ligand on the outer surface of the carrier. Accordingly, the carrier binds to the surface of a desired tissue or cell in the brain parenchyma via the exposed second ligand.

In the second embodiment, the relationship between the chain length of the aforementioned first non-charged segment and the chain length of the aforementioned second non-charged segment may be any. To efficiently expose the GLUT1 ligand on the outer surface of the carrier before cleavage of the aforementioned bond and to efficiently expose the second ligand on the outer surface of the carrier after cleavage of the aforementioned bond, the chain length of the aforementioned first non-charged segment is preferably set to be substantially the same as the chain length of the aforementioned second non-charged segment, or longer than the chain length of the aforementioned second non-charged segment. In one embodiment, in the carrier of the present invention, the chain length of the first non-charged segment is set to be longer than the chain length of the second non-charged segment so that the second molecule is sufficiently enveloped in the first block copolymer (e.g., of the second molecule contained in the carrier of the present invention, the proportion of the second molecule presented on the carrier surface is, for example, less than 50% (preferably less than 40%, less than 30%, less than 20%, less than 10%, less than 5% or less than 1%). The length of the first non-charged segment necessary for enveloping the second molecule varies depending on the size of the second molecule. Therefore, it is difficult to unconditionally specify the difference in the lengths of the first non-charged segment and the second non-charged segment by a numerical value. However, for example, when the second molecule is a low molecule (molecular weight of not more than 1000) and the non-charged segment is liner PEG, it is estimated that the second molecule can be enveloped by the first block copolymer when the first non-charged segment is longer by not less than 5 KDa, preferably not less than 10 KDa, in the number average molecular weight than the second non-charged segment. When the first non-charged segment is other than liner PEG, a difference in the length corresponding to the difference in the molecular weight of PEG suffices.

Specific examples of the first and the second block copolymers and a production method thereof are explained below.

While a case where the complex-forming segment is a charged segment is exemplarily shown, the present invention is not limited thereto. When the complex-forming segment is a hydrophobic segment, the first and the second block copolymers can be produced in the same manner as in the charged segment.

First, the unmodified and uncleavable first block copolymer, and the unmodified second block copolymer (hereinafter sometimes to be abbreviated as unmodified block copolymer) are explained.

The unmodified block copolymer can be divided into an embodiment in which the charged segment is an anionic segment (hereinafter sometimes to be abbreviated as unmodified anionic block copolymer) and an embodiment in which the charged segment is a cationic segment (hereinafter sometimes to be abbreviated as unmodified cationic block copolymer).

Examples of the unmodified anionic block copolymer include the following block copolymers.

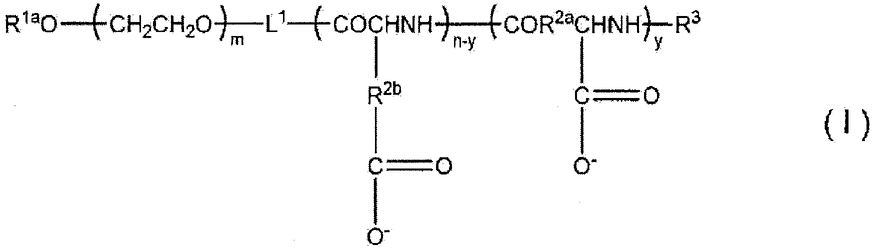

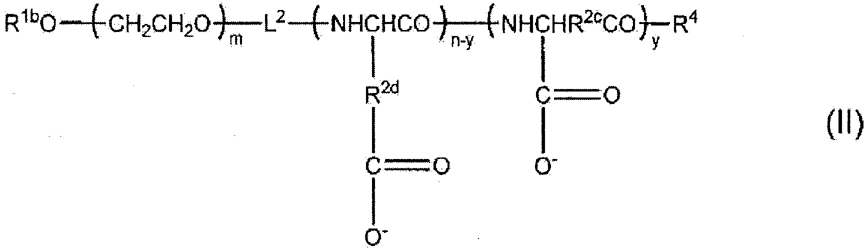

In the structural formulas of the formulas (I) and (II), a segment having a repeat unit number (degree of polymerization) of "m" is PEG-derived non-charged hydrophilic segment (hereinafter sometimes to be referred to "PEG segment"), and a segment comprising a portion with a repeat unit number of "n-y" and a portion with "y" in combination is polyanion-derived anionic segment (hereinafter sometimes to be indicated as "polyanion segment").

In the formulas (I) and (II), wherein R^{1a} and R^{1b} are each independently a hydrogen atom or an unsubstituted or substituted liner or branched C₁₋₁₂ alkyl group. Examples of the liner or branched C₁₋₁₂ include methyl, ethyl, n-propyl, iso-propyl, n-butyl, sec-butyl, tert-butyl, n-pentyl, n-hexyl, decyl, undecyl and the like. As the substituent when substituted, acetalized formyl group, cyano group, formyl group, carboxyl group, amino group, C₁₋₆ alkoxycarbonyl group, C₂₋₇ acylamido group, the same or different tri-C₁₋₆ alkylsiloxy group, siloxy group or silylamino group can be mentioned. Acetalization means formation of an acetal moiety by a reaction between carbonyl of formyl and two molecules of alkanol having a carbon number of 1 - 6 or optionally branched alkylenediol having a carbon number of 2 - 6. It is also a protection method of the carbonyl group. For example, when the substituent is an acetalized formyl group, it can be hydrolyzed under mild acidic conditions and converted to another substituent formyl group (-CHO) (or aldehyde group).

In the formulas (I) and (II), L¹ and L² are each a linker group. Specifically, L¹ is preferably -(CH₂)_{b}-NH- (wherein b is an integer of 1 - 5), and L² is preferably -(CH₂)_{c}-CO- (wherein c is an integer of 1 - 5).

In the formulas (I) and (II), R^{2a}, R^{2b}, R^{2c} and R^{2d} represent each independently a methylene group or an ethylene group. When both R^{2a} and R^{2b} are methylene groups, the polymer corresponds to poly(aspartic acid derivative), when they are ethylene groups, the polymer corresponds to poly(glutamic acid derivative), when both R^{2c} and R^{2d} are methylene groups, the polymer corresponds to poly(aspartic acid derivative), and when they are ethylene groups, the polymer corresponds to poly(glutamic acid derivative). In these formulas, when R^{2a} and R^{2b} (R^{2b} and R^{2a}) represent both methylene group and ethylene group, and when R^{2c} and R^{2d} (R^{2d} and R^{2c}) represent both methylene group and ethylene group, the repeat units of the aspartic acid derivative and glutamic acid derivative may, respectively, form a block or present randomly.

In the formulas (I) and (II), R³ is a hydrogen atom, a protecting group, a hydrophobic group or a polymerizable group. Specifically, R³ is preferably acetyl group, acryloyl group or methacryloyl group.

In the formulas (I) and (II), R⁴ is a hydroxyl group, an oxybenzyl group, an -NH-(CH₂)ₐ-X group or an initiator residue. Here, a is an integer of 1 - 5, and X is preferably an amine compound residue containing 1 kind or 2 kinds or more of primary, secondary, tertiary amine and quaternary ammonium salt, or a compound residue other than amine. In some cases, R⁴ is preferably -NH-R⁹ (wherein R⁹ is an unsubstituted or substituted liner or branched C₁₋₂₀ alkyl group).

In the formulas (I) and (II), m is an integer of 5 - 2,000, preferably an integer of 5 - 270, more preferably an integer of 10 - 100. In addition, n is an integer of 2 - 5,000, y is an integer of 0 - 5,000, n and y is preferably an integer of 5 - 300, more preferably an integer of 10 - 100. Note that y is not larger than n.

While the respective repeat units in the formulas (I) and (II) are shown in a particular order for the convenience of description, respective repeat units can be present in a random order. Particularly, only the respective repeat units in the polyanion segment can be preferably present in a random order as mentioned above.

While the molecular weight (Mw) of the block copolymers shown by the formulas (I) and (II) is not limited, it is preferably 3,000 - 30,000, more preferably 5,000 - 20,000. As for individual segment, the molecular weight (Mw) of the PEG segment is preferably 500 - 15,000, more preferably 1,000 - 5,000, and the molecular weight (Mw) of the polyanion segment as a whole is preferably 500 - 50,000, more preferably 1,000 - 20,000.

While the production method of the block copolymers shown by the formulas (I) and (II) is not limited, a method including, for example, synthesizing a segment (PEG segment) containing R^{1a}O- or R^{1b}O- and a block portion of PEG chain in advance, sequentially polymerizing given monomers at one of the terminals of the PEG segment (a terminal opposite from R^{1a}O- or R^{1b}O-), and, where necessary, substituting or converting the side chain to contain an anionic group, or a method including synthesizing the above-mentioned PEG segment and a block portion having a side chain containing an anionic group in advance, and linking them with each other and the like can be mentioned. The methods and conditions for various reactions in the production methods can be appropriately selected or determined in consideration of a conventional method. The above-mentioned PEG segment can be prepared, for example, using the production methods of PEG segment portion of the block copolymers described in WO96/32434, WO96/33233 and WO97/06202 and the like.

As a more specific production method of the block copolymers shown by the formulas (I) and (II), a method including synthesizing a block copolymer using a PEG segment derivative having an amino group in the terminal and polymerizing, at the amino terminal, N-carboxy anhydride (NCA) of protected amino acid such as β-benzyl-L-aspartate (BLA), Nε-Z-L-lysine and the like, and then substituting or converting the side chain of each segment so that a side chain has the aforementioned anionic group can be preferably mentioned.

In the present invention, specific examples of the block copolymers shown by the formulas (I) and (II) preferably include, an anionic block copolymer of the following formula (hereinafter sometimes to be referred to "PEG-P(Asp)") comprising polyethylene glycol (hereinafter sometimes to be referred to "PEG") as a non-charged segment, and polyaspartic acid (hereinafter sometimes to be referred to "P(Asp)") as an anionic segment and the like (in the following formulas, Na⁺ is shown as an example of counter cation, but the counter cation is not limited to these).

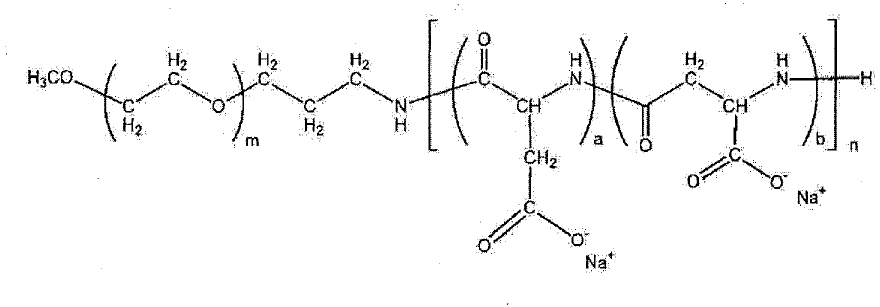

In the above-mentioned formula,
m is an integer showing the degree of polymerization of PEG,
n is an integer showing the degree of polymerization of P(Asp), and
a, b are each a number greater than 0 and less than 1, provided that a+b=1.

As PEG-P(Asp), one showing a molecular weight (Mw) of PEG segment: 2,000, and a unit number (n in the above-mentioned formula) of P(Asp) showing the polyanion segment: 70 or 75 is particularly preferable.

On the other hand, preferable examples of the unmodified cationic block copolymer include those represented by the following formula (III) and/or (IV).

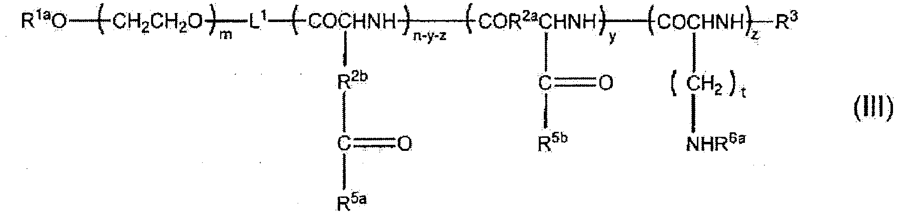

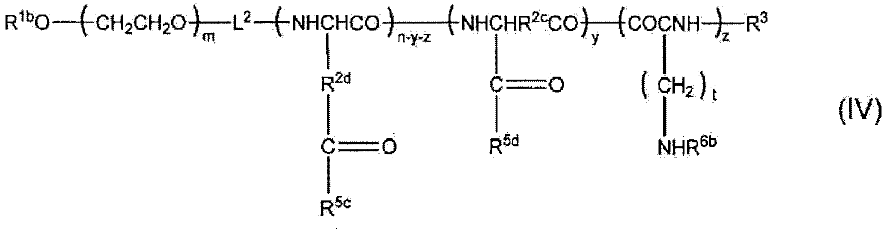

In the structural formulas of the formulas (III) and (IV), a segment with a repeat unit number (degree of polymerization) of "m" is a PEG-derived non-charged hydrophilic segment (PEG segment), and a segment comprising a portion with a repeat unit number of "n-y-z", a portion with "y", and a portion with "z" in combination is a polycation-derived cationic segment (hereinafter polycation segment).

In the formulas (III) and (IV), R^{1a} and R^{1b} are each independently a hydrogen atom or an unsubstituted or substituted liner or branched C₁₋₁₂ alkyl group. Examples of the liner or branched C₁₋₁₂ include methyl, ethyl, n-propyl, iso-propyl, n-butyl, sec-butyl, tert-butyl, n-pentyl, n-hexyl, decyl, undecyl and the like. As the substituent when substituted, acetalized formyl group, cyano group, formyl group, carboxyl group, amino group, C₁₋₆ alkoxycarbonyl group, C₂₋₇ acylamido group, the same or different tri-C₁₋₆ alkylsiloxy group, siloxy group or silylamino group can be mentioned. Acetalization means formation of an acetal moiety by a reaction between carbonyl of formyl and two molecules of alkanol having a carbon number of 1 - 6 or optionally branched alkylenediol having a carbon number of 2 - 6. It is also a protection method of the carbonyl group. For example, when the substituent is an acetalized formyl group, it can be hydrolyzed under mild acidic conditions and converted to another substituent formyl group (-CHO: or aldehyde group).

In the formulas (III) and (IV), L¹ and L² are each a linker group. Specifically, L¹ is preferably -(CH₂)_{b}-NH-(wherein b is an integer of 1 - 5), and L² is preferably-(CH₂)_{c}-CO- (wherein c is an integer of 1 - 5).

In the formulas (III) and (IV), R^{2a}, R^{2b}, R^{2c} and R^{2d} are each independently a methylene group or an ethylene group. When both R^{2a} and R^{2b} are methylene groups, the polymer corresponds to poly(aspartic acid derivative), when they are ethylene groups, the polymer corresponds to poly(glutamic acid derivative), when both R^{2c} and R^{2d} are methylene groups, the polymer corresponds to poly(aspartic acid derivative), and when they are ethylene groups, the polymer corresponds to poly(glutamic acid derivative). In these formulas, when R^{2a} and R^{2b} (R^{2b} and R^{2a}) represent both methylene group and ethylene group, and when R^{2c} and R^{2d} (R^{2d} and R^{2c}) represent both methylene group and ethylene group, the repeat units of the aspartic acid derivative and glutamic acid derivative may form a block or present randomly.

In the formulas (III) and (IV), R³ is a hydrogen atom, a protecting group, a hydrophobic group or a polymerizable group. Specifically, R³ is preferably acetyl group, acryloyl group or methacryloyl group.

In the formulas (III) and (IV), R⁴ is a hydroxyl group, an oxybenzyl group, an -NH-(CH₂)ₐ-X group or an initiator residue. Here, a is an integer of 1 - 5, and X is preferably an amine compound residue containing 1 kind or 2 kinds or more of primary, secondary, tertiary amine, quaternary ammonium salt or guanidino group, or a compound residue other than amine. In some cases, R⁴ is preferably -NH-R⁹ (wherein R⁹ is an unsubstituted or substituted liner or branched C₁₋₂₀ alkyl group).

In the formulas (III) and (IV), R^{5a}, R^{5b}, R^{5c} and R^{5d} are each independently a hydroxyl group, am oxybenzyl group or an-NH-(CH₂)ₐ-X group. Here, a is an integer of 1 - 5, and X is preferably an amine compound residue containing 1 kind or 2 kinds or more of primary, secondary, tertiary amine, quaternary ammonium salt or guanidino group, or a compound residue other than amine.

In the total number of R^{5a} and R^{5b} and the total number of R^{5c} and R^{5d}, at least two -NH-(CH₂)ₐ-X groups (wherein X is (NH(CH₂)₂)ₑ-NH₂ (e is an integer of 0 - 5)) are preferably present, more preferably the -NH-(CH₂)ₐ-X group is present in not less than 50% of the above-mentioned total number, further preferably not less than 85% of the above-mentioned total number.

In addition, all or a part of R^{5a}, R^{5b}, R^{5c} and R^{5d} are/is preferably an -NH-(CH₂)ₐ-X group (wherein a is 2, and X is (NH(CH₂)₂)ₑ-NH₂ (e is 1)).

In the above-mentioned -NH-(CH₂)ₐ-X group recited as an example of R⁴ as well as R^{5a}, R^{5b}, R^{5c} and R^{5d}, X is particularly preferably selected from the groups represented by the following formulas.

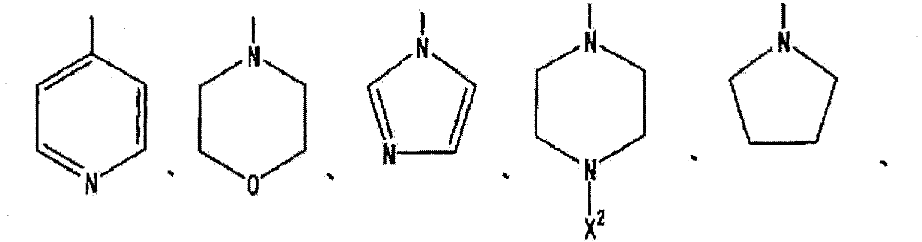

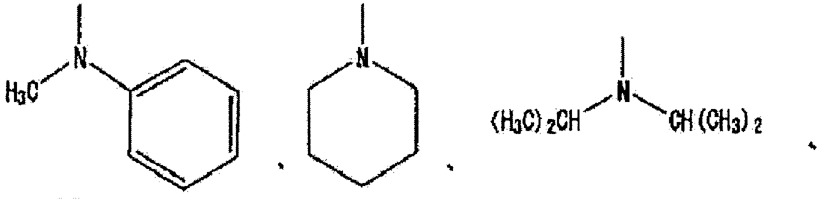

-(CH₂)_{f}-NH₂, -(NR^{7a}(OH₂)_{d1})ₑ₁-NHR^{6a}, -N(CH₃)₂,

-N(CH₂CH₃)₂, -(NR^{7b}(CH₂)_{d2})ₑ₂-(NR^{7c}(CH₂)_{d3})ₑ₃-NHR^{8b},

-(CH₂)_{g}CH₃,

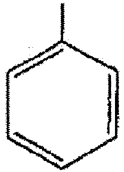

or

-NHC(=NH)NH₂

In each of the above-mentioned formulas, X² is a hydrogen atom or a C₁₋₆ alkyl group or an amino C₁₋₆ alkyl group, R^{7a}, R^{7b} and R^{7c} are each independently a hydrogen atom or a methyl group, d1, d2 and d3 are each independently an integer of 1 - 5, e1, e2 and e3 are each independently an integer of 1 - 5, f is an integer of 0 - 15, g is an integer of 0 - 15, R^{8a} and R^{8b} are each independently a hydrogen atom or a protecting group. The protecting group is preferably a group selected from the group consisting of Z group, Boc group, acetyl group and trifluoroacetyl group, which are generally used as amino-protecting groups.

In the formulas (III) and (IV), R^{6a} and R^{6b} are each independently a hydrogen atom, -C(=NH)NH₂, or a protecting group. The protecting group is preferably a group selected from the group consisting of Z group, Boc group, acetyl group and trifluoroacetyl group, which are generally used as amino-protecting groups. In the formulas (III) and (IV), t is preferably an integer of 2 - 6, more preferably 3 or 4.

In the formulas (III) and (IV), m is an integer of 5 - 2,000, preferably an integer of 5 - 270, more preferably an integer of 10 - 100. In addition, n is an integer of 2 - 5,000, y is an integer of 0 - 5,000, and z is an integer of 0 - 5,000. n is an integer of 5 - 300, more preferably an integer of 0 or 10 - 100. y and z are each preferably an integer of 0 or 5 - 300, more preferably an integer of 0 or 10 - 100. The total of y and z (y+z) is not larger than n.

While the respective repeat units in the formulas (III) and (IV) are shown in a particular order for the convenience of description, respective repeat units can be present in a random order. Particularly, only the respective repeat units in the polycationic segment can be preferably present in a random order as mentioned above.

While the molecular weight (Mw) of the block copolymers shown by the formulas (III) and (IV) is not limited, it is preferably 23,000 - 45,000, more preferably 28,000 - 34,000. As for individual segment, the molecular weight (Mw) of the PEG segment is preferably 500 - 15,000, more preferably 1,000 - 5,000, and the molecular weight (Mw) of the polycationic segment as a whole is preferably 500 - 50,000, more preferably 1,000 - 30,000.

While the production method of the block copolymers shown by the formulas (III) and (IV) is not limited, a method including, for example, synthesizing a segment containing R^{1a}O- or R^{1b}O- and a block portion of PEG chain (PEG segment) in advance, sequentially given monomers are polymerized at one of the terminals of the PEG segment (terminal opposite from R^{1a}O- or R^{1b}O-), and, where necessary, substituting or converting the side chain to contain a cationic group; or a method including synthesizing the above-mentioned PEG segment and a block portion having a side chain containing a cationic group in advance, and linking them with each other and the like can be mentioned. The methods and conditions for various reactions in the production methods can be appropriately selected or determined in consideration of a conventional method. The above-mentioned PEG segment can be prepared, for example, using the production methods of PEG segment portion of the block copolymers described in WO96/32434, WO96/33233 and WO97/06202 and the like.

As a more specific production method of the block copolymers shown by the formulas (III) and (IV), a method including synthesizing a block copolymer using a PEG segment derivative having an amino group in the terminal and polymerizing, at the amino terminal, N-carboxy anhydride (NCA) of a protected amino acid such as β-benzyl-L-aspartate (BLA), Nε-Z-L-lysine and the like, and then substituting or converting, with diethylenetriamine (DET) etc., the side chain of each segment so that a side chain has the aforementioned cationic group can be preferably mentioned.

On the other hand, for the cleavable first block copolymer, the non-charged segment and the charged segment may be linked via a desired cleavable bond when they are linked in the above-mentioned respective steps. As such linking means, various known methods may be appropriately selected according to the kind of the cleavable bond. For example, when a disulfide bond is introduced as a cleavable bond, the non-charged segment and the charged segment may be linked using a bifunctional reagent containing a disulfide bond such as H₂N-R^{10a}-S-S-R^{10b}-NH₂ (wherein R^{10a} and R^{10b} are the same or different and each is a liner or branched chain alkylene group having a carbon number of 1 - 10, preferably 1 - 6, more preferably 1, 2, 3 or 4) (e.g., H₂N-C₂H₅-S-S-C₂H₅-NH₂) and the like. The following scheme shows an embodiment using polyethylene glycol as the non-charged segment and poly(Asp-AP) as the charged segment, and those of ordinary skill in the art can easily understand that it is also applicable to non-charged segments and charged segments other than these. For example, a non-charged polymer having a carboxyl group introduced into the terminal (e.g., PEG-COOH) is obtained by reacting a non-charged polymer having an amino group introduced into the terminal (e.g., PEG-NH₂) with succinic anhydride to substitute the terminal amino group with -NH-CO-(CH₂)₂-COOH.

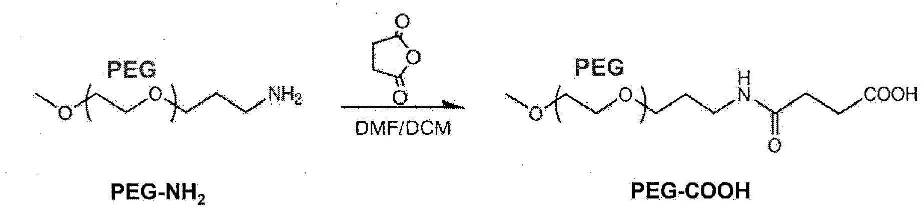

Next, a polymerization initiator having an amino group at the terminal and having a non-charged segment and a disulfide bond in the molecule (e.g., PEG-SS-NH₂) can be obtained by reacting the non-charged polymer having a carboxyl group at the terminal (e.g., PEG-COOH) with cistamine dihydrochloride and 1-ethyl-3-(3-dimethylaminopropyl)carbodiimide (EDC) hydrochloride.

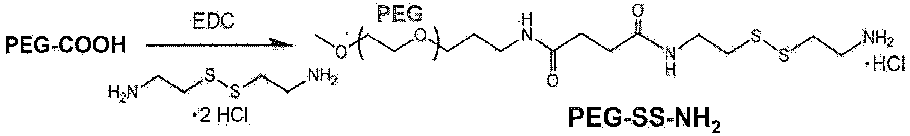

Then, monomers constituting a desired charged polymer (e.g., polyanion, polycation) are sequentially polymerized at the amino acid terminal of the obtained polymerization initiator, and then, if necessary, the side chain is substituted or converted to contain an anionic group or cationic group. For example, when polyaspartic acid or a derivative thereof is linked, a polymerization reaction occurs by adding NCA-BLA to the polymerization initiator (e.g., PEG-SS-NH₂) obtained above, whereby the aforementioned block copolymer (e.g., PEG-SS-PBLA) in which the non-charged segment and poly(β-benzyl-L-aspartate) (i.e., PBLA) are linked via a disulfide bond can be obtained. Furthermore, this block copolymer is reacted with 1,5-diaminopentane to introduce a cationic group aminopentane (AP) into the side chain of aspartic acid, whereby the aforementioned block copolymer (e.g., PEG-SS-P(Asp-AP)) in which the non-charged segment and poly(Asp-AP) are linked via a disulfide bond can be obtained.

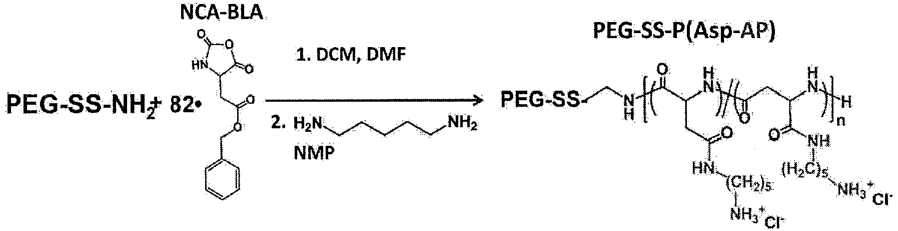

As a result of such reaction series, block copolymers of the formulas (I), (II), (III) and (IV) wherein L¹ and L² are substituted by a linker having a disulfide bond (e.g., -HN-R^{10a}-S-S-R^{10b}-NH- (e.g., -HN-C₂H₅-S-S-C₂H₅-NH-)) can be obtained.

When a bond cleavable at pH 6.5 or below is introduced as a cleavable bond, the non-charged segment and the charged segment may be linked according to the method described in, for example, Proc Natl Acad Sci U S A. 2015 Oct 6; 112(40):12486-91. As a result, block copolymers of the formulas (I), (II), (III) and (IV) wherein L¹ and L² are the linker represented by 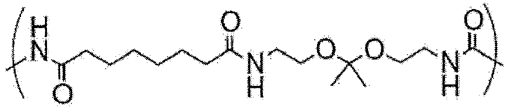 can be obtained.

The modification of the first block copolymer with the GLUT1 ligand can be performed by a method well known to those of ordinary skill in the art. As one embodiment of such method, an example of a preparation method of a polymer modified by glucose (particularly, Glc(6)-PEG-poly(anion) block copolymer or Glc(6)-PEG-poly(cation) block copolymer) is explained below. The Glc(6)-PEG-poly(anion) block copolymer or Glc(6)-PEG-poly(cation) block copolymer can be obtained by, for example, protecting hydroxyl groups on carbons other than position 6 in glucose and polymerizing the block copolymer with the glucose (WO 2015/075942).

In Scheme 1A, a synthesis Scheme of a compound of the formula (I) wherein n₁ is 44 and m₁ is 80 is shown as an example.

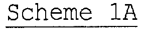

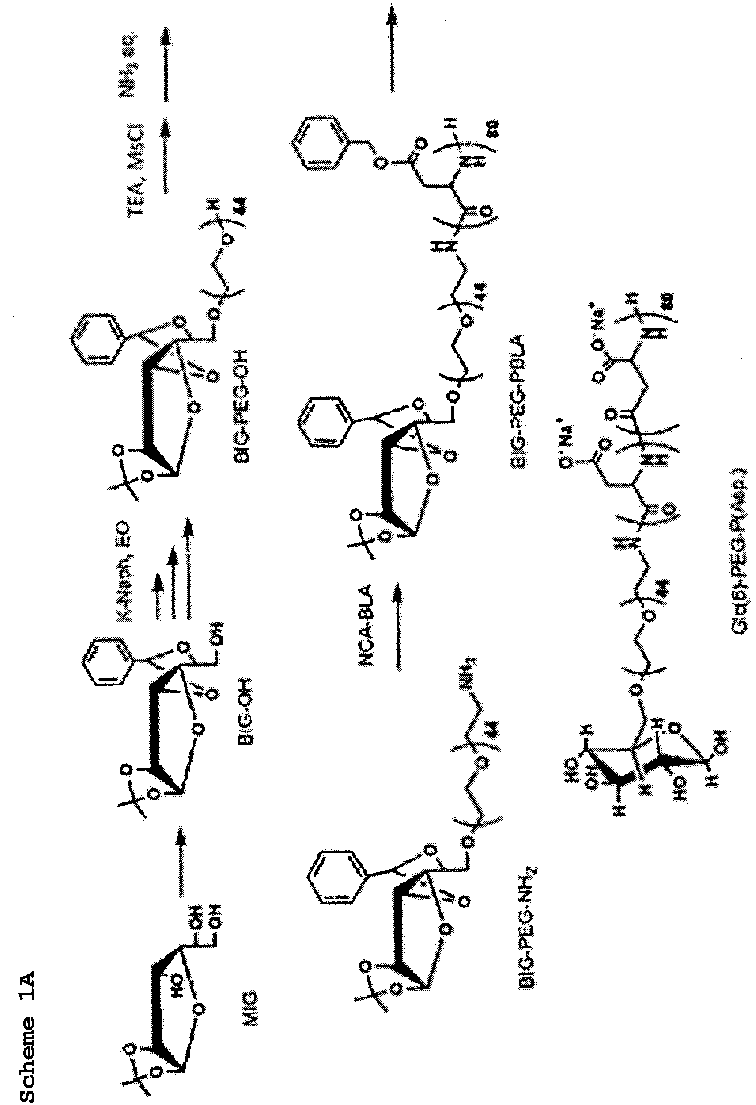

In Scheme 1A, EO is ethylene oxide; K-Naph is potassium naphthalene; TEA is triethylamine; MsCl is methane sulfonylchloride; NH₃aq. is aqueous ammonia; and NCA-BLA is β-benzyl-L-aspartate-N-carboxy anhydride.

Scheme 1A is explained briefly below. The introduction of a protecting group into glucose can be achieved with, for example, 1,2-O-isopropylidene 5,6-O-benzylidene-α-D-glucofuranose (hereinafter to be referred to as "BIG"). For example, in the case of preparing PIC micelle or PICsome, ethylene oxide is polymerized at BIG to synthesize BIG-PEG-OH. BIG is obtained by, for example, protecting OH groups as substituents on the carbon atoms at position 3 and position 5 of 1,2-0-isopropylidene-α-D-glucofuranose (hereinafter to be referred to as "MIG") with benzyl groups. To be specific, BIG is obtained by reacting MIG with benzaldehyde and extracting with ethyl acetate. Then, to make the molecular weight of PEG constant, BIG-OH is, before polymerization reaction, preferably freeze-dried over benzene in a reaction vessel and dried under reduced pressure (e.g., dried under reduced pressure at 70°C overnight), thereby attaching BIG-OH to the wall of the vessel. The degree of polymerization can be appropriately adjusted by the amount of ethylene oxide to be added. After the polymerization, the OH group of BIG-PEG-OH is aminated to give BIG-PEG-NH₂. A polycation or polyanion, or a protected precursor thereof (e.g., β-benzyl-L-aspartate-N-carboxy anhydride (BLA-NCA), which is a protected monomer of polyaspartic acid, or γ-benzyl-L-glutamate-N-carboxy anhydride (BLG-NCA), which is a protected monomer of polyglutamic acid) can be further polymerized at the NH₂ group of BIG-PEG-NH₂ to give BIG-PEG-poly(anion) or BIG-PEG-poly(cation). The degree of polymerization can be appropriately adjusted by the amount of the polycation or polyanion, or a protected precursor thereof. Finally, the protecting groups in the glucose and the anion or cation can be deprotected to give glucose-PEG-poly(anion) or glucose-PEG-poly(cation). The copolymer conjugated with glucose can be used for preparing PIC micelle or PICsome. To be specific, PIC micelle or PICsome can be spontaneously formed by mixing a polymer having a polycation block and a polymer having a polyanion block in an aqueous solution at a ratio that neutralizes electric charge. In this way, a PIC micelle or PICsome in which the polyion complex is covered with a biocompatible moiety which is modified with glucose can be obtained.

Similarly, Glc(3)-PEG-poly(anion) and Glc(3)-PEG-poly(anion) can be synthesized in totally the same way as above except that, for example, 1,2,5,6-di-O-isopropylidene-α-D-glucofuranose (DIG) is used instead of BIG as a starting material (see Scheme 1B). Also, similarly, Glc(2)-PEG-poly (anion) and Glc(2)-PEG-poly (anion) can also be synthesized appropriately by those of ordinary skill in the art.

In Scheme 1B, a synthesis scheme of a compound of the formula (X) wherein n₁ is 44 and m₁ is 80, is shown as an example. Scheme 1B is the same as Scheme 1A except that DIG is used instead of BIG as a starting material.

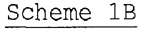

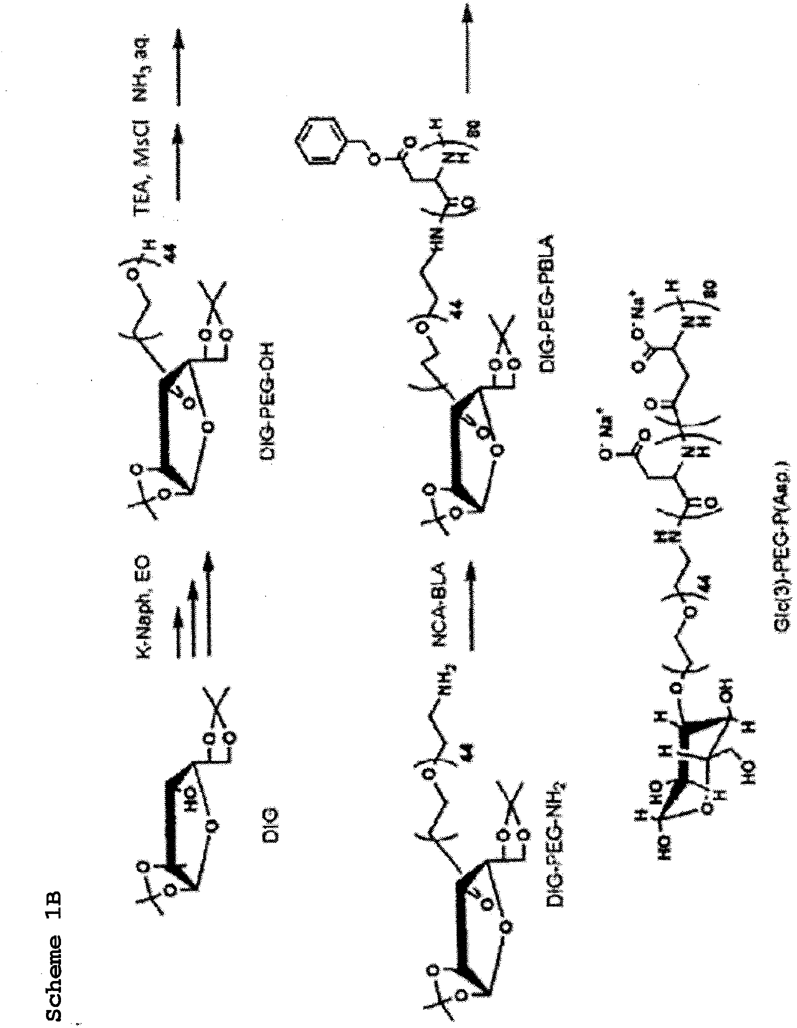

In Scheme 1B, EO is ethylene oxide; K-Naph is potassium naphthalene; TEA is triethylamine; MsCl is methanesulfonyl chloride; NH₃aq. is aqueous ammonia; and NCA-BLA is β-benzyl-L-aspartate-N-carboxy anhydride.

Modification of the second block copolymer with the second molecule can also be performed by selecting various methods well known to those of ordinary skill in the art according to the kind of the second molecule. The second molecule and the second block copolymer can be chemically bonded directly or indirectly, namely, via or not via a group derived from a crosslinking agent (linker).

As a manner of a direct bond between the second molecule and the second block copolymer, -COO- [obtained by, for example, forming ester bond between carboxyl group in the second molecule and hydroxyl group in the second non-charged segment (e.g., PEG)], -O- (obtained by, for example, forming ether bond between hydroxyl group in the second molecule and hydroxyl group in the second non-charged segment (e.g., PEG)), -CONH-(obtained by, for example, forming amide bond between carboxyl group in the second molecule and amino group introduced into the second non-charged segment (e.g., PEG)), -CH=N- (obtained by, for example, forming Schiff bond between aldehyde group in the second molecule and amino group introduced into the second non-charged segment (e.g., PEG)), -CH₂NH- (obtained by further reducing the Schiff bond), -NH- and the like can be mentioned.

When direct binding is not available for the chemical binding between the second molecule and the second block copolymer, a known condensing agent, an activator to activate functional group in the second molecule or the second block copolymer (preferably, the second non-charged segment (e.g., PEG)), divalent crosslinking agent and the like can also be used. Examples of the condensing agent and activator include carbodiimides [for example, N,N'-dicyclohexylcarbodiimide, 1-ethyl-3-(3-dimethylaminopropyl)carbodiimide (EDC) etc.], succinimides [for example, N-hydroxysuccinimide, N-hydroxysulfosuccinimide (NHSS), dibenzylcyclooctyne-N-succinimide ester (DBCO-NHS ester) etc.], sodium azide and the like. Examples of the divalent crosslinking agent include homoreactive crosslinking agent and heteroreactive crosslinking agent. Examples of the homoreactive one include dimethyl adipinimidate, disuccinimidyl suberate and the like, and examples of the heteroreactive one include succinimidyl-3-(2-pyridyldithio)propionate, N-(6-maleimidocaproyloxy)succinimide, N-succinimidyl-6-maleimidehexanoate and the like.

As an example, when an aspartic acid ligand is introduced as the second molecule, the desired aspartic acid ligand can be introduced by, for example, using an aspartic acid-introducing reagent of the following structure and the like having aspartic acid having carboxyl group protected with ethyl group and terminal alkynyl group, reacting the terminal alkynyl group of the reagent with the terminal N₃ group of the second block copolymer having N₃ group at the terminal on the non-charged segment side to introduce the carbon group-protected aspartic acid ligand into the terminal at the non-charged segment side, and deprotecting the carboxyl group.

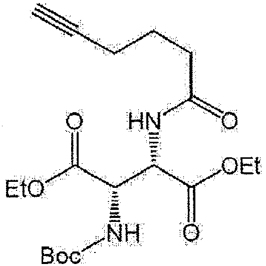

As another example, DBCO is introduced into amino group in aspartic acid having carboxyl group protected with alkyl group (e.g., ethyl group) by using DBCO-NHS ester. The desired aspartic acid ligand can be introduced by subjecting the DBCO to Click reaction with the terminal N₃ group of the second block copolymer having N₃ group at the terminal on the non-charged segment side to introduce carbon group-protected aspartic acid ligand into the terminal at the non-charged segment side, and deprotecting the carboxyl group.

The carrier of the present invention can be prepared by mixing the first block copolymers entirely or partly modified with GLUT1 ligand and the second block copolymers entirely or partly modified with the second molecule, which were prepared by the above steps.

As the embodiment of the carrier of the present invention, vesicles such as micelle, liposome, PICsome and the like, dendrimer, nanosphere and hydrogel for drug delivery can be mentioned. In the present invention, the advantage of the use of the carrier for drug delivery is that, for example, the drug concentration at the target site is increased by encapsulating a drug in the inside of the carrier, or side effects of the drug in the parts other than the target site are reduced. The carrier used in the present invention is not particularly limited, but the average particle size (diameter) thereof is, for example, not more than 400 nm, not more than 200 nm, not more than 150 nm, not more than 100 nm or not more than 80 nm, and for example, not less than 20 nm, not less than 30 nm or not less than 40 nm. The carrier used in the present invention has an average particle size of, for example, 30 nm - 150 nm or, for example, 30 nm - 100 nm.

In the present invention, the average particle size of the carrier can be measured using a commercially available dynamic light scattering (DLS) measuring apparatus.

As the micelle used in the present invention, micelle for drug delivery can be mentioned. As a micelle for drug delivery, a micelle formed with a block copolymer is known. A block copolymer for forming a micelle is not particularly limited. In the case of a polyion complex micelle (PIC micelle), a copolymer of charged polymer block (e.g., polyanion block or polycation block) and non-charged (biocompatible) block (e.g., polyethylene glycol block) or a pharmaceutically acceptable salt thereof is available. As the block copolymer, a biodegradable block copolymer is preferably used. As such copolymer, various copolymers are known and any of them can be used in principle. For example, as a biodegradable block copolymer having high biocompatibility, polyethylene glycol-polyaspartic acid, polyethylene glycol-polyglutamic acid, polyethylene glycol-poly((5-aminopentyl)-aspartic acid) block copolymers and the like can be used. As PIC micelle, a micelle having a polyion complex layer formed by an electrostatic interaction between polyanion and polycation is known. To stabilize hydrophobic portions in the inside of the micelle, each charged block may be linked with a hydrophobic portion such as cholesteryl group and the like at a terminal different from PEG forming an outer shell (e.g., see siRNA micelle in Example). For labeling of block copolymer with a fluorescence dye, the terminal opposite from the polyethylene glycol side of the block copolymer may be modified with a fluorescence dye.

As polyion complex polymersome used in the present invention, PICsome for drug delivery can be mentioned. As the PICsome for drug delivery, PICsome formed with a block copolymer is known. As the block copolymer for forming PICsome, a combination of a block copolymer of non-charged block (e.g., PEG block) and polycation block and homopolyanion, a combination of a block copolymer of non-charged block (e.g., PEG block) and polyanion block and homopolycation and the like can be mentioned. In the present invention, the PICsome can be formed by using the aforementioned PEG block etc. and polycation block or polyanion block as non-charged segments and charged segments of the first and the second block copolymers (in the case of the cleavable first block copolymer, a cleavable bond is interlaid between the non-charged block (e.g., PEG block) and charged block (polycation block or polyanion block)). As the block copolymer, a biodegradable block copolymer is preferably used. As such copolymer, various copolymers are known and any of them can be used in principle. For example, as a biodegradable block copolymer having high biocompatibility, poly(aspartic acid-tetraethylenepentaamine (Asp-TEP)) block copolymer and polyethylene glycol-poly((5-aminopentyl)-aspartic acid) block copolymer can be used. In PICsome, GLUT1 ligand and the second molecule are respectively linked to the terminal on the first non-charged segment side of the first block copolymer and the terminal on the second non-charged segment side of the second block copolymer, and the chain lengths of the first non-charged segment and the second non-charged segment are adjusted, whereby GLUT1 ligand and the second molecule can be exposed on the outer surface of the PICsome.

While the liposome to be used in the present invention is not particularly limited, a liposome formed from phospholipid, for example, dimyristoylphosphatidylcholine (DMPC) can be mentioned. Various liposomes have been known for a long time, and those of ordinary skill in the art can appropriately prepare liposomes. Those of ordinary skill in the art can appropriately encapsulate a drug in a liposome.

Vesicles can be formed using the above-mentioned polymers by a well-known method. Generally, vesicles can be obtained by stirring a solution of the above-mentioned first and second block copolymers dissolved at a concentration not less than a given concentration. In addition, vesicles formed based on a polyion complex (PIC micelle, PICsome etc.) can be obtained by mixing a polymer having a polycation portion and a polymer having a polyanion portion at the same ratio.

For example, when the second charged segment is charged oppositely to the electric charge of the first charged segment (that is, either one is a cationic segment and the other is an anionic segment), a polyion complex is formed between the cationic segment and the anionic segment in the both block copolymers by mixing the first block copolymer and the second block copolymer to neutralize the electric charge, and vesicles (PIC micelle, PICsome etc.) formed based on the polyion complex can be obtained. A polyion complex can also be formed and a vesicle (PIC micelle, PICsome etc.) formed based on the polyion complex can also be obtained by adding a cationic polymer (e.g., homopolycation) and/or an anionic polymer (e.g., homopolyanion) in addition to the first block copolymer and the second block copolymer and mixing them to neutralize the electric charge of the whole of the first charged segment, the second charged segment, and the cationic polymer (e.g., homopolycation) and/or the anionic polymer (e.g., homopolyanion). When the first charged segment and the second charged segment have the same electric charge (that is, both are cationic segment or anionic segment), a polyion complex can also be formed and vesicle (PIC micelle, PICsome etc.) formed based on a polyion complex can also be obtained by adding a cationic polymer (e.g., homopolycation) or anionic polymer (e.g., homopolyanion) charged oppositely to the first and second charged segments in addition to the first block copolymer and the second block copolymer and mixing them to neutralize the electric charge of the whole of the first charged segment, the second charged segment, and cationic polymer (e.g., homopolycation) or anionic polymer (e.g., homopolyanion). In one embodiment, the cationic polymer or the anionic polymer to be added in addition to the first block copolymer and the second block copolymer is the aforementioned unmodified block copolymer. The length of the non-charged segment in the additional unmodified block copolymer is preferably substantially the same as or shorter than the length of a shorter non-charged segment from the first and the second non-charged segments. A method of encapsulating a drug in a vesicle is well known to those of ordinary skill in the art, and a well-known method can also be used in the present invention. For example, to encapsulate a drug in a PIC micelle, the drug may be added to a micelle solution after micelle formation. The drug is spontaneously encapsulated in the PIC micelle due to the electric charge thereof. In the case of PICsome, for example, a drug can be encapsulated in PICsome by preparing a mixture of a polymer forming the PICsome and the drug and stirring and mixing them. In the case of liposome, a drug can also be encapsulated by preparing a mixture of a polymer forming the liposome and stirring and mixing them. It is also possible to crosslink anionic block and cationic block contained in the polyion complex. While the crosslinking agent used for this object is not particularly limited, for example, 1-ethyl-3-(3-dimethylaminopropyl)carbodiimide hydrochloride (EDC) capable of condensing amino group and carboxy group is preferably used.

When the proportion of the GLUT1 ligand-conjugated polymer in the block copolymer in which a non-charged segment is exposed on the outside of the carrier, from among the block copolymers forming vesicles, is not less than 10 mol% and less than 40 mol%, the delivery efficiency of the composition to the brain parenchyma is particularly high. The proportion of the GLUT1 ligand-conjugated polymer in the above-mentioned block copolymer in which a non-charged segment is exposed on the outerside of the carrier may be not less than 10 mol% and less than 40 mol%, preferably 20 - 30 mol%, more preferably 22 - 28 mol%, further preferably 24 - 26 mol% (e.g., about 25 mol%). When the proportion of the GLUT1 ligand-conjugated polymer in the above-mentioned block copolymer in which a non-charged segment is exposed on the outside of the carrier is not less than 40%, the delivery efficiency of the composition to the brain vascular endothelial cell is particularly high. Particularly, the proportion of the GLUT1 ligand-conjugated polymer in the above-mentioned block copolymer in which a non-charged segment is exposed on the outside of the carrier may be set to 40 - 100 mol%, for example, 40 - 80 mol%, for example, 40 - 60 mol%.

The above-mentioned block copolymer in which a non-charged segment is exposed on the outside of the carrier is defined as follows. That is, when the chain length of the first non-charged segment is longer than the chain length of the second non-charged segment, the second non-charged segment is generally enveloped in the first non-charged segment, the first block copolymer corresponds to the block copolymer in which a non-charged segment is exposed on the outside of the carrier. On the other hand, when the chain length of the first non-charged segment is substantially the same as the chain length of the second non-charged segment, both the first and second block copolymers correspond to the block copolymer in which a non-charged segment is exposed on the outside of the carrier. However, even when the chain length of the second non-charged segment is substantially the same as the chain length of the first non-charged segment or longer than the chain length of the first non-charged segment, when the first block copolymer is rigid and the second block copolymer is flexible, the first block copolymer sometimes takes a structure enveloping the second block polymer. In this case, the first block copolymer corresponds to the block copolymer in which a non-charged segment is exposed on the outside of the carrier.

In one embodiment, the chain length of the first non-charged segment is longer than the chain length of the aforementioned second non-charged segment, and not less than 10 mol% and less than 40 mol% (preferably 20 - 30 mol%, more preferably 22 - 28 mol%, further preferably 24 - 26 mol% (e.g., about 25%)) of the aforementioned first block copolymer is modified with GLUT1 ligand. In this embodiment, the first block copolymer may be an uncleavable form. As mentioned above, when many glucose molecules modify the carrier, the efficiency of dissociation of the carrier from vascular endothelial cells decreases when the carrier is transported from the vascular endothelial cells to the brain parenchyma, and the ratio of the carriers that reach the brain parenchyma may decrease. Therefore, a decrease in the efficiency of the dissociation of the carrier from the vascular endothelial cells can be avoided and the transport efficiency of the carrier into the brain parenchyma can be increased by setting the proportion of the first block copolymer modified by the GLUT1 ligand to about not less than 10 mol% and less than 40 mol% of the whole first block copolymer.

In one embodiment, the chain length of the first non-charged segment is substantially the same as the chain length of the aforementioned second non-charged segment, and the proportion of the first block copolymer modified by GLUT1 ligand is not less than 10 mol% and less than 40 mol% (preferably 20 - 30 mol%, more preferably 22 - 28 mol%, further preferably 24 - 26 mol% (e.g., about 25 mol%)) of the total of the first block copolymer and the aforementioned second block copolymer. In this embodiment, the first block copolymer may be an uncleavable form. A decrease in the efficiency of the dissociation of the carrier from the vascular endothelial cells can be avoided and the efficiency of transport of the carrier into the brain parenchyma can be increased by setting the proportion of the first block copolymer modified by the GLUT1 ligand to about not less than 10 mol% and less than 40 mol% of the total of the first block copolymer and the aforementioned second block copolymer.

In one embodiment, the chain length of the first non-charged segment is longer than the chain length of the second non-charged segment, and 40 - 100 mol% (preferably, 60 - 100 mol%, more preferably 80 - 100 mol%, further preferably 90 - 100 mol%) of the first block copolymer is modified with GLUT1 ligand. In this embodiment, the first block copolymer may be a cleavable form. Using the cleavable first block polymer, a moiety including the GLUT1 ligand is cleaved from the carrier after transported into the endosome in the brain vascular endothelial cell or brain parenchyma. Therefore, even when the proportion of the first block copolymer modified by the GLUT1 ligand is increased up to 40 - 100 mol% of the whole first block copolymer, the carrier is efficiently dissociated from the vascular endothelial cells and can be transported into the brain parenchyma. In addition, the efficiency of transport from the blood to the brain vascular endothelial cells can be enhanced by increasing the proportion of the first block copolymer modified by the GLUT1 ligand up to 40 - 100 mol% of the whole first block copolymer.

In one embodiment, the chain length of the first non-charged segment is substantially the same as the chain length of the aforementioned second non-charged segment, and the proportion of the first block copolymer modified with GLUT1 ligand is 40 - 99 mol% (preferably, 60 - 99 mol%, more preferably 80 - 99 mol%, further preferably 90 - 99 mol%) of the total of the first block copolymer and the second block copolymer. In this embodiment, the first block copolymer may be a cleavable form. Using the cleavable first block polymer, a moiety including the GLUT1 ligand is cleaved from the carrier after transported into the endosome in the brain vascular endothelial cell or brain parenchyma. Therefore, even when the proportion of the first block copolymer modified by the GLUT1 ligand is increased up to 40 - 99 mol% of the total of the first block copolymer and the second block copolymer, the carrier is efficiently dissociated from the vascular endothelial cells and can be transported into the brain parenchyma. In addition, the transport efficiency from the blood to the brain vascular endothelial cells can be enhanced by increasing the proportion of the first block copolymer modified by the GLUT1 ligand up to 40 - 99 mol% of the total of the first block copolymer and the second block copolymer.

In the prepared carrier, the orientation of the first non-charged segment and the orientation of the second non-charged segment are preferably toward the outside of the carrier, and the orientation of the first non-charged segment and the orientation of the second non-charged segment are preferably the same. As mentioned above, when the chain length of the second non-charged segment is substantially the same as the chain length of the first non-charged segment or longer than the chain length of the first non-charged segment, the first block copolymer is rigid, and the second block copolymer is flexible, the first non-charged segment is oriented toward the outside of the carrier whereas the second non-charged segment is bent inside the carrier so that the first block copolymer preferably takes a structure to envelope the second block polymer. In this case, therefore, the orientation of the first non-charged segment and the orientation of the second non-charged segment are preferably different.

When the first block copolymer takes a structure to envelope the second block polymer, it is expected that the second ligand is protected in the blood and a decrease in the blood retention property and a decrease in the BBB passage efficiency due to the second ligand are prevented.

In one embodiment, the carrier of the present invention contains the first block copolymer and the second block copolymer, wherein
the first block copolymer is a block copolymer of the first polyethylene glycol modified with the first molecule and the first charged segment, and has a disulfide bond between the first polyethylene glycol and the first charged segment,
the second block copolymer is a block copolymer of the second polyethylene glycol modified with the second molecule and the second charged segment, and
the first molecule and the second molecule are different.

In this embodiment, preferably, the first molecule is glucose.

In this embodiment, preferably, the second molecule is at least one kind selected from the group consisting of a low-molecular-weight compound, a peptide, a protein and an antibody. The second molecule can bind to the target molecule on the target cell surface in the brain tissue (preferably in the brain parenchyma).

In this embodiment, preferably, the chain length of the first polyethylene glycol is longer than the chain length of the second polyethylene glycol.

In this embodiment, preferably, the first block copolymer envelopes the second block copolymer.

In this embodiment, preferably, the first polyethylene glycol and the second polyethylene glycol have the same orientation.

In this embodiment, preferably, the second charged segment is charged oppositely to the electric charge of the first charged segment.

In this embodiment, at least one, preferably both, of the first charged segment and the second charged segment is/are polyamino acid.

In one embodiment, at least one of the first charged segment and the second charged segment is polyaspartic acid.

In one embodiment, at least one of the first charged segment and the second charged segment is poly((5-aminopentyl)-aspartic acid).

In one embodiment, one of the first charged segment and the second charged segment is polyaspartic acid and the other is poly((5-aminopentyl)-aspartic acid).

In a new aspect, the present invention also provides a carrier containing the first block copolymer and the second block copolymer, wherein
the first block copolymer is a block copolymer of the first non-charged segment and the first charged segment, and at least some proportion of the first block copolymer is modified with the first molecule,
the second block copolymer is a block copolymer of the second non-charged segment and the second charged segment,
the first molecule is GLUT1 ligand,
the first block copolymer has a bond cleavable at pH 6.5 or below between the first non-charged segment and the first charged segment,
the second block copolymer has a bond cleavable at pH 6.5 or below between the aforementioned second non-charged segment and the aforementioned second charged segment, and one or both of the first charged segment and the second charged segment is/are poly(Asp-DET) or poly(Glu-DET). The definition of each term is the same as that in the aforementioned carrier of the present invention except that at least some proportion of the second block copolymer does not require to be modified with the second molecule, and has a bond cleavable at pH 6.5 or below between the second non-charged segment and the second charged segment. The carrier of this embodiment is, due to the effect of the GLUT1 ligand, selectively incorporated into the endosome of the brain vascular endothelial cells, and dissociated from GLUT1 when the bonds cleavable at pH 6.5 or below and present between the first non-charged segment and the first charged segment and between the second non-charged segment and the second charged segment are cleaved under the acidic environment in the endosome, whereby the charged segment is exposed on the carrier surface. Since poly(Asp-DET) (or poly(Glu-DET)) has membrane-damaging property under the acidic environment in the endosome, the endosomal escape in the brain vascular endothelial cells is promoted. Therefore, the carrier of this embodiment is useful as a carrier for selective delivery of a drug to brain vascular endothelial cells.

### 3. Composition

The present invention also provides a composition containing the above-mentioned carrier and a drug encapsulated in the carrier. The composition is preferably a composition for delivery of the drug in the brain, and among others, a composition for passing the drug through the blood-brain barrier, blood-nerve barrier, blood-retinal barrier or blood-cerebrospinal fluid barrier and/or selectively delivering the drug to a desired site in the brain. Specific examples of the desired tissue or cell in the brain include, but are not particularly limited, hippocampus or cerebellum as a site in the brain parenchyma, neuron, astrocyte, microglia, vascular endothelial cell and the like as a cell in the brain parenchyma. It is also possible to expect, for example, further species-selective transport among the neurons. Such composition for selective delivery of the drug to a desired site in the brain can be constituted using a carrier which is selectively transported to a desired tissue or cell in the brain. The detail of such carrier is as explained above. While a preparation method of the composition of the present invention is not particularly limited, for example, a method including mixing the carrier of the present invention to make the drug to be carried in the carrier, a method including mixing the first block copolymers entirely or partly modified by the GLUT1 ligand and the second block copolymers entirely or partly modified by the second molecule, which constitute the carrier of the present invention, with the drug to form the carrier of the present invention and simultaneously make the drug to be carried in the carrier and the like can be used.

While the drug to be used in present invention is not particularly limited, bioactive substance, antibody, nucleic acid, biocompatible fluorescence dye, and contrast agents such as a contrast agent for ultrasonication, MRI and CT and the like can be used. In the present invention, a drug can be highly selectively delivered to the brain, and highly selectively delivered to a desired site (e.g., vascular endothelial cell, particular tissues or cells in the brain parenchyma). Therefore, while the drug is not particularly limited, for example, a bioactive substance enhancing the physiological function of the brain, a bioactive substance capable of treating a brain disease, an antibody recognizing an antigen characteristic of a brain disease, a nucleic acid controlling expression of a gene related to a brain disease, a biocompatible fluorescence dye capable of staining the brain, and a contrast agent such as a contrast agent for ultrasonication, MRI and CT and the like can be used. For example, the composition of the present invention using, as a drug, a bioactive substance enhancing the physiological function of the brain, a bioactive substance capable of treating a brain disease, an antibody recognizing an antigen characteristic of a brain disease or a nucleic acid controlling expression of a gene related to a brain disease can be provided as a pharmaceutical composition. The composition of the present invention using, as a drug, a biocompatible fluorescence dye capable of staining the brain or a contrast agent such as a contrast agent for ultrasonication, MRI and CT and the like can be provided as a diagnostic reagent.

According to the present invention, a therapeutic or prophylactic drug for a brain disease can be used as the drug. In this case, according to the present invention, a method for the treatment or prophylaxis of a brain disease comprising administering a carrier for drug delivery having an outer surface modified with GLUT1 ligand and encapsulating a therapeutic or prophylactic drug for the brain disease to a subject in need thereof according to an administration schedule is provided. Similarly, according to the present invention, a method for the treatment or prophylaxis of a peripheral neurological disease comprising administering a carrier for drug delivery having an outer surface modified with GLUT1 ligand and encapsulating a therapeutic or prophylactic drug for the peripheral neurological disease to a subject in need thereof according to an administration schedule is provided. Similarly, according to the present invention, a method for the treatment or prophylaxis of a retinal disease comprising administering a carrier for drug delivery having an outer surface modified with GLUT1 ligand and encapsulating a therapeutic or prophylactic drug for the retinal disease to a subject in need thereof according to an administration schedule is provided. In one embodiment, the administration schedule in the present invention includes administering the composition to a fasted or hypoglycemia-induced subject. In one embodiment, the administration schedule in the present invention includes administering the composition to a fasted or hypoglycemia-induced subject and inducing an increase in the blood glucose level in the subject.

According to the present invention, therefore, a pharmaceutical composition for the treatment or prophylaxis of a brain disease containing a therapeutic or prophylactic drug for the brain disease is provided. It is obvious that, according to the present invention, the uptake of drugs into the brain is improved and the pharmaceutical composition of the present invention is useful for the treatment or prophylaxis of the brain diseases. According to the present invention, a pharmaceutical composition for the treatment or prophylaxis of a peripheral neurological disease containing a therapeutic drug or prophylactic drug for the peripheral neurological disease is provided. It is obvious that, according to the present invention, the uptake of drugs into the peripheral nerve is improved and the pharmaceutical composition of the present invention is useful for the treatment or prophylaxis of the peripheral neurological disease. According to the present invention, furthermore, a pharmaceutical composition for the treatment or prophylaxis of a retinal disease containing a therapeutic drug or prophylactic drug for the retinal disease is provided. It is obvious that, according to the present invention, the uptake of drugs into the retina is improved and the pharmaceutical composition of the present invention is useful for the treatment or prophylaxis of the retinal disease. According to the present invention, the above-mentioned therapeutic or prophylactic drug in the form of being encapsulated in the carrier can be contained in the composition.

As the brain disease, brain diseases which can be treated by allowing a therapeutic drug for the brain disease to pass through the blood-brain barrier, for example, anxiety, depression, sleep disorder, Alzheimer's disease, Parkinson's disease and multiple sclerosis and the like can be mentioned. Therefore, in the present invention, therapeutic drugs or prophylactic drugs for brain diseases such as antianxiety agents, antidepressants, sleep-inducing agents, therapeutic drugs for Alzheimer's disease, therapeutic drugs for Parkinson's disease, therapeutic drugs for multiple sclerosis and the like may be used to treat these brain diseases. As a therapeutic drug for Alzheimer's disease, Aβ antibody is well known, as a therapeutic drug for Parkinson's disease, dopamine receptor agonist and L-DOPA are well known, and as a therapeutic drug for multiple sclerosis, corticosteroid drug, interferon β (IFNβ), and immunosuppressants are well known, and these therapeutic drugs can be used in the present invention. As the peripheral neurological disease, peripheral nerve diseases which can be treated by allowing a therapeutic drug for peripheral neurological diseases to pass through the blood-brain barrier, for example, Guillain-Barre syndrome, Fisher syndrome and chronic inflammatory demyelinating polyradiculoneuropathy can be mentioned. As the retinal disease, retinal diseases which can be treated by allowing a therapeutic drug for retinal diseases to pass through the blood-brain barrier, for example, retinitis pigmentosa, gyrate atrophy of choroid and retina, choroideremia, crystallin retinopathy, congenital amaurosis, congenital stationary night blindness, Oguchi's disease, fundus albipunctatus, punctata albescens retinopathy, pigmented paravenous retinochoroidal atrophy, Stargardt disease, vitelliform macular dystrophy, juvenile retinoschisis, central areolar choroidal dystrophy, occult macular dystrophy, familial exudative vitreoretinopathy and stria pigmentosa retinae can be mentioned.

When a charged segment is used as a complex-forming segment, the carrier of the present invention is advantageous for delivering a hydrophilic drug. For example, when at least one, preferably both, of the first charged segment and the second charged segment is/are polycation, it is advantageous for the delivery of nucleic acid, negative-charged protein and peptide and other hydrophilic drugs. When a hydrophobic segment is used as a complex-forming segment, the carrier of the present invention is advantageous for delivering a hydrophobic drug.

According to the previous findings by the present inventors, a carrier having outer surface modified by GLUT1 ligand is accumulated in the brain by merely administering the carrier. Therefore, an administration schedule using the carrier of the present invention and a composition encapsulating a drug therein does not require induction of hypoglycemia by fasting and the like and/or induction of an increase in the blood glucose level. According to the previous findings by the present inventors, when a carrier having its outer surface modified by GLUT1 ligand such that the GLUT1 ligand is exposed on the surface, specifically vesicles such as micelle or a polyion complex polymersome (PICsome) and the like is administered according to an administration schedule, these carriers are remarkably delivered into the brain (brain parenchyma) across the blood-brain barrier. Therefore, in one embodiment, the administration schedule using the carrier of the present invention and a composition encapsulating a drug therein include administering the composition to a fasted or hypoglycemia-induced subject. In one embodiment, the administration schedule using the carrier of the present invention and a composition encapsulating a drug therein includes administering the composition to a fasted or hypoglycemia-induced subject and inducing an increase in the blood glucose level in the subject. In one embodiment, in the administration schedule using the carrier of the present invention and a composition encapsulating a drug therein, the composition may be administered to a subject simultaneously with, or continuously or sequentially with the induction of an increase in the blood glucose level in the subject. The administration schedule may or may not have an interval between the administration of the composition to a subject and induction of an increase in the blood glucose level in the subject. When the composition is administered simultaneously with the induction of an increase in the blood glucose level in the subject, the composition may be administered in the form of a mixture with a drug causing induction of an increase in the blood glucose level to the subject, or administered in a form different from the drug causing induction of an increase in the blood glucose level in the subject. When the composition is administered continuously or sequentially with the induction of an increase in the blood glucose level in the subject, the composition may be administered before or after the induction of an increase in the blood glucose level in the subject, preferably, the composition can be administered before induction of an increase in the blood glucose level in the subject. When an increase in the blood glucose level is induced in the subject before administration of the composition to the subject, the composition is preferably administered to the subject within 1 hr, 45 min, 30 min, 15 min or 10 min from induction of an increase in the blood glucose level in the subject. When an increase in the blood glucose level is induced in the subject after administration of the composition to the subject, the increase in the blood glucose level in the subject is preferably induced within 6 hr, 4 hr, 2 hr, 1 hr, 45 min, 30 min, 15 min or 10 min from administration of the composition to the subject. The above-mentioned administration schedule may be performed not less than 2 cycles. The anteroposterior relation between glucose administration and sample administration can be determined by the timing of passage through the blood-brain barrier.

Cerebral cortex is constituted of 6 layers, and a molecular layer (layer 1), an external granular layer (layer 2), an external pyramidal layer (layer 3), an internal granular layer (layer 4), an internal pyramidal layer (layer 5) and a multiform layer (layer 6) exist from the surface layer. According to the present invention, the carrier can be delivered to the brain parenchyma through any of these layers. Of these layers, the delivery of the carrier by the present invention is remarkably effective particularly in the external pyramidal layer (layer 3) and internal granular layer (layer 4).

In the present invention, a large carrier (diameter about 30 nm - 100 nm) such as micelle or PICsome effectively passes through the blood-brain barrier efficiently and can be delivered highly selectively to a desired site in the brain (e.g., vascular endothelial cell or particular tissue or cell in the brain parenchyma).

The carrier or composition of the present invention can be directly administered to a subject, or can also be administered according to a particular administration schedule explained below. In such administration schedule, in one embodiment, the subject is fasted or the subject is induced to have hypoglycemia, after which the composition is administered to the subject. In the administration schedule according to the present invention, in one embodiment, the subject is fasted or the subject is induced to have hypoglycemia, after which the composition is administered to the subject and an increase in the blood glucose level is induced in the subject. In the administration schedule according to the present invention, administration of the composition to the subject is performed simultaneously with, or continuously or sequentially with the induction of an increase in the blood glucose level in the subject. Induction of hypoglycemia is considered to be useful for expressing GLUT1 on an inner surface of vascular endothelial cells (e.g., brain vascular endothelial cells). According to the present invention, however, an increase in the blood glucose level is extremely effective for the delivery of the carrier or composition of the present invention to the brain in the administration subject. According to the previous findings by the present inventors, when the blood concentration of the carrier or composition of the present invention in the fasted or hypoglycemia-induced subject is a given value or higher, an increase in the blood glucose level causes extremely effective delivery of the carrier or composition of the present invention into the brain. The carrier or composition of the present invention can be delivered into the brain of the subject for a while even after an increase in the blood glucose level is induced in the subject.

To maintain the concentration of the carrier or composition of the present invention in the blood at a certain level or above, the carrier or composition of the present invention is preferably administered to the subject by infusion. In this way, even a carrier or composition having a short blood retention time can easily ensure a given blood concentration. For example, effects may be easily enhanced by administering siRNA micelle encapsulating siRNA having a short blood retention time by infusion to the subject. Infusion administration can be preferably performed for not less than 10 min, not less than 15 min, not less than 30 min, not less than 45 min, not less than 60 min, not less than 90 min or not less than 2 hr. Infusion administration is preferably performed at a constant infusion rate. For example, administration at a constant infusion rate is possible by using a precise administration pump. The infusion administration may be performed simultaneously with the induction of an increase in the blood glucose level in the subject, or an increase in the blood glucose level may be induced during infusion administration.

When the carrier or composition of the present invention is administered according to the administration schedule of the present invention, the delivery efficiency to the brain is selectively enhanced. Therefore, the carrier or composition of the present invention can be used for delivering a drug to the brain. Particularly, the carrier or composition of the present invention can be used for delivering a drug selectively to a desired site in the brain (e.g., vascular endothelial cell and particular tissue or cell in the brain parenchyma). The carrier or composition of the present invention can also make a drug pass through the blood-brain barrier. Therefore, the carrier or composition of the present invention can be used for delivering drugs such as bioactive substance, antibody, nucleic acid, biocompatible fluorescence dye, and a contrast agent such as a contrast agent for ultrasonication, MRI and CT and the like, and the like, to the brain parenchyma to which the delivery has conventionally been difficult. Particularly, the carrier or composition of the present invention can be used for delivering drugs such as bioactive substance, antibody, nucleic acid, biocompatible fluorescence dye, and a contrast agent such as a contrast agent for ultrasonication, MRI and CT and the like, and the like, selectively to a particular tissue or cell in the brain parenchyma. The carrier or composition of the present invention can make a drug accumulated in brain vascular endothelial cells. Therefore, the carrier or composition of the present invention can be used for delivering drugs such as bioactive substance, antibody, nucleic acid, biocompatible fluorescence dye, and a contrast agent such as a contrast agent for ultrasonication, MRI and CT and the like, and the like, selectively to brain vascular endothelial cells to which the delivery has conventionally been difficult. In addition, the carrier or composition of the present invention can also be used for delivering a drug for weakening or destroying the adhesion between brain vascular endothelial cells to the brain vascular endothelial cells. Similarly, the carrier or composition of the present invention can be used for delivering drugs such as bioactive substance, antibody, nucleic acid, biocompatible fluorescence dye, and a contrast agent such as a contrast agent for ultrasonication, MRI and CT and the like, and the like, to retina, peripheral nerve and/or cerebrospinal fluid. The carrier or composition of the present invention can also be used for delivering drugs such as bioactive substance, antibody, nucleic acid, biocompatible fluorescence dye, and a contrast agent such as a contrast agent for ultrasonication, MRI and CT and the like, and the like, to vascular endothelial cells respectively present in the blood-nerve barrier, blood-retinal barrier or blood-cerebrospinal fluid barrier. The carrier or composition of the present invention can also be used for delivering a drug for weakening or destroying the adhesion between vascular endothelial cells to the vascular endothelial cells respectively present in the blood-nerve barrier, blood-retinal barrier or blood-cerebrospinal fluid barrier. By weakening or destroying the adhesion between vascular endothelial cells, the barrier function can be weakened and various drugs can pass through the barriers.

The carrier or composition of the present invention can be administered orally or parenterally (e.g., intravenous administration or intraperitoneal administration).

According to the present invention, the carrier can encapsulate drugs such as bioactive substance, antibody, nucleic acid, biocompatible fluorescence dye, and a contrast agent such as a contrast agent for ultrasonication, MRI and CT and the like, and can effectively deliver the drug encapsulated in the carrier to brain, peripheral neural tissue, retina and/or cerebrospinal fluid.

When the carrier or composition of the present invention is administered to a subject, the carrier or composition passes through the blood-brain barrier and enter into the brain due to the action of GLUT1 ligand modifying the first block copolymer. Thereafter, by the action of the second molecule ligand modifying the second block copolymer, the carrier or composition moves selectively to a desired site in the brain (e.g., vascular endothelial cell or particular tissue or cell in the brain parenchyma). Here, when the cleavable first block copolymer is used, a cleavable bond that links the first non-charged segment and the first complex-forming segment is specifically cleaved at a desired site in the brain where a carrier is to be selectively transported or a site to be passed through before reaching a desired site in the brain where a carrier is to be selectively transported (e.g., under environment conditions in the brain parenchyma or endosome in vascular endothelial cell). Thus, the first non-charged segment is removed, and the second non-charged segment modified with the second molecule ligand is efficiently exposed on the outside of the carrier. As a result, a drug carried in the composition of the present invention can be selectively delivered to a desired site in the brain. Such delivery method of a drug using the composition of the present invention is also provided by the present invention.

### [Examples]

### [Experimental Example 1] Production of PEG-SS-P(Asp-AP)

### (1) Synthesis of PEG-COOH

First, PEG-NH₂ (molecular weight of PEG 12kDa, 800 mg, 0.0667 mmol, NOF CORPORATION) was dissolved in dichloromethane (16 mL, Nakarai Tesque, Inc.), succinic anhydride (140 mg, 0.714 mmol, Tokyo Chemical Industry Co., Ltd.) dissolved in DMF (4 mL, Nakarai Tesque, Inc.) was added, and the mixture was reacted at 35°C. The reaction solution was subjected to a dialysis treatment using a dialysis membrane with a fraction molecular weight 6-8 kDa (Spectra/Por, Funakoshi Co., Ltd.) and lyophilized to give a white powder (yield 94%). This was analyzed by ¹H-NMR (JEOL Ltd.) and ion exchange column chromatography. As a result, it was confirmed that the reaction proceeded quantitatively and all terminal amino groups were converted to a carboxyl group (PEG-COOH) (Fig. 5).

### (2) Synthesis of PEG-SS-NH₂

Next, PEG-COOH (500 mg, 0.0407 mmol) was dissolved in pure water (10 mL), the pH was adjusted to around neutral (6.5 - 7.5), cistamine dihydrochloride (183 mg, 0.976 mmol, Tokyo Chemical Industry Co., Ltd.) and 1-ethyl-3-(3-dimethylaminopropyl)carbodiimide (EDC) hydrochloride (156 mg, 1.05 mmol, Tokyo Chemical Industry Co., Ltd.) were added as powders, and the mixture was reacted at room temperature for 12 hr. The reaction solution was subjected to a dialysis treatment using a dialysis membrane with fraction molecular weight 6-8kDa (Spectra/Por, Funakoshi Co., Ltd.) (outer liquid 0.01% HCl aq) and lyophilized to give a white powder (yield 94%). This was analyzed by ¹H-NMR (JEOL Ltd.) and ion exchange column chromatography. As a result, it was found that the reaction proceeded quantitatively and, as mentioned above, a polymerization initiator having a disulfide bond in a molecule (PEG-SS-NH₂•HCl) was obtained (Fig. 6).

### (3) Synthesis of PEG-SS-P(Asp-AP)

PEG-SS-NH₂•HCl was dissolved again in pure water, subjected to a dialysis treatment using a dialysis membrane with a fraction molecular weight of 6-8 kDa (outer liquid 0.01% aqueous ammonia solution), and lyophilized to give PEG-SS-NH₂. Using this as an initiator and according to a previous report, polymerization of NCA-BLA was performed. To be specific, PEG-SS-NH₂ (94 mg, 0.076 mmol) was dissolved in dichloromethane (0.7 mL, Kanto Chemical Co., Inc.), NCA-BLA (152 mg, 0.600 mmol) dissolved in a mixed solvent of DMF (0.3 mL, Kanto Chemical Co., Inc.) and dichloromethane (2 mL, Kanto Chemical Co., Inc.) was added and the mixture was reacted at 35°C for 2 days. This reaction solution was added dropwise to hexane:ethyl acetate=2:3 (volume ratio, both Godo Co., Ltd.), and the precipitate was recovered by suction filtration. This was dried under reduced pressure overnight to give PEG-SS-PBLA. Next, PEG-SS-PBLA (200 mg, 0.0069 mmol) was dissolved in NMP (10 mL, Nakarai Tesque, Inc.), and the mixture was cooled to 5°C. On the other hand, 1,5-diaminopentane (6 mL, Tokyo Chemical Industry Co., Ltd.) was similarly dissolved in NMP (6 mL, Nakarai Tesque, Inc.), and the mixture was cooled to 5°C, and the polymer solution was added dropwise. The mixture was reacted at 5°C for 1 hr. The reaction solution was subjected to a dialysis treatment using a dialysis membrane with a fraction molecular weight of 15 kDa (Spectra/Por, Funakoshi Co., Ltd.) (outer liquid 0.01% HCl aq), and lyophilized to give a white powder (yield 89%, average degree of polymerization 72). This was analyzed by ¹H-NMR (JEOL Ltd.) and size exclusion column chromatography. As a result, cleavage was observed in about 10% of S-S bond but a novel polymer (PEG-SS-P(Asp-AP)) having a disulfide bond between PEG and the charged segment was obtained almost quantitatively (Fig. 7).

### [Experimental Example 2] Evaluation of responsiveness of PEG-SS-P(Asp-AP) to reductive environment

To evaluate responsiveness of the novel polymer (PEG-SS-P(Asp-AP)) having a disulfide bond between PEG and the charged segment, which was synthesized in Experimental Example 1, to the reductive environment, it was dissolved in 10 mM phosphate buffer (pH=7.4) at 1 mg/mL, a DTT solution was added to provide an equivalent reductive environment to the glutathione concentration (3 mM) in the brain, and the mixture was stirred by pipetting and left standing. The solution was sampled 5 min later, 15 min later and 50 min later, respectively, and changes in the molecular weight were evaluated by HPLC. As a result, it was suggested that the ratio of the decomposed fractions PEG-SH (20 min), Sh-P(Asp-Ap) (22 min) increased with the progress of time, and almost all S-S bonds were cleaved 50 min later (Fig. 8).

### [Experimental Example 3] Preparation of micelle having S-S bond

A block copolymer (PEG-PAsp) comprising PEG (molecular weight 2kDa) and a charged segment having opposite electric charge (negative charge chain this time), which was shorter than the novel polymer (PEG-SS-P(Asp-AP)) having a disulfide bond between PEG and the charged segment, which was synthesized in Experimental Example 1 was synthesized according to a previous report. These two kinds of polymers were each dissolved in 10 mM phosphate buffer at 1 mg/mL, mixed at balanced point of electric charge, and stirred at room temperature for 2 min to give a micelle covered with two kinds of PEGs having different lengths. Thereafter, a condensing agent EDC was dissolved in pure water at 1-10 mg/mL, and the same amount thereof was added to the micelle solution to immobilize the core portion of the micelle (crosslinking). These micelles were each evaluated by dynamic light scattering measurement (Zetasizer, Malvern Instruments Ltd) to find that monodispersed particles having an average particle size of about 50 nm were obtained (Fig. 9). The same amount of a reducing agent dithiothreitol (DTT) (Wako Pure Chemical Industries, Ltd.) solution (5 mM) was added to these micelle solutions, and the mixture was stood at room temperature for 2 hr, and dynamic light scattering measurement was performed similarly. As a result, when the EDC concentration was 1 mg/mL or less, immobilization of the micelle core portion was not sufficient, and change of form was confirmed along with the elimination of PEG. On the other hand, when 3-10 mg/mL EDC solution was added, the core was sufficiently immobilized, and a decrease in the particle size along with the elimination of PEG was confirmed while maintaining the shape of the micelle (Fig. 9).

### [Experimental Example 4] Evaluation of responsiveness of micelle having S-S bond to reductive environment

From among the micelles prepared in Experimental Example 3, a micelle solution added with10 mg/mL EDC expected to have most strongly immobilized core portion was used to evaluate the responsiveness to the reductive environment in detail. DTT solutions were prepared by diluting DTT with 10 mM phosphate buffer based on the reduction potential to achieve a reducing power corresponding to Glutathione concentration of 0.02 - 6 mM. The same amount of DTT solution was added to respective micelle solutions, and change of particle size of micelle was evaluated by dynamic light scattering measurement. As a result, it was confirmed that in the region (1-3 mM) corresponding to the glutathione concentration in the brain, the particle size decreased (Fig. 10). This suggests PEG with molecular weight of 12kDa was eliminated along with the cleavage of disulfide bond, and 2kDa PEG alone remained on the surface layer. This hypothesis is also supported by the fact that micelle having 2kDa PEG on the surface layer has a particle size of about 30 nm. Furthermore, in a region (0.01 mM) corresponding to the glutathione concentration in blood, the particle size did not change. Thus, this micelle has a possibility of exhibiting a function to maintain the structure thereof stably in blood and eliminate PEG only in the brain parenchyma after passing through BBB (Fig. 10).

### [Experimental Example 5] Production of PIC micelle (carrier A) composed of Glc-PEG_{(2K)}-P(Asp-AP) and (Boc-Asp)-PEG-PAsp

In this Experimental Example, PIC micelle containing the uncleavable first block polymer modified by glucose ligand, Glc-PEG_{(2K)}-P(Asp-AP) and, and the second block copolymer modified by Boc-amino group-modified aspartic acid, (Boc-Asp)-PEG(_{2K})-PAsp was produced.

### (1) Synthesis of Glc-PEG_{(2K)}-P(Asp-AP)

1,2-O-Isopropylidene-α-D-glucofranose (10.13 g) was transferred into a flask, pyridine (60 mL) was added, then dichloromethane (60 mL) was added and the mixture was completely dissolved. Thereafter, pivaloyl chloride (5.5 mL) was added dropwise, and the mixture was stirred at room temperature for 5 hr. The reaction solution was concentrated under reduced pressure, pure water was added to precipitate the resultant product and it was recovered by filter filtration. Successively, methanol warmed to 65°C in an oil bath at 65°C was added by small portions. When completely dissolved, the power of the oil bath was turned off, and the mixture was allowed to cool to room temperature. Furthermore, the mixture was transferred to a refrigerator at 4°C and the resultant product was precipitated, and the resultant product MIG-Piv (9.25 g) was recovered by suction filtration. MIG-Piv (9.25 g) was transferred to a flask, Acetone Dimethyl Acetal (230 mL) was added and then TsOH_{•}H₂O (292 mg) was added. This was refluxed at 75°C for 30 min, cooled to room temperature, trimethylamine (1 mL) was added dropwise, and then toluene (50 mL) was added. This was concentrated under reduced pressure, toluene (50 mL) was further added, and, similarly, concentration and addition of toluene were repeated 3 times. Thereafter, the resultant product was extracted with dichloromethane, dehydrated over sodium sulfate, suction filtered, dried under reduced pressure and recovered. This was purified by silica gel column to recover the resultant product DIG-Piv (10.4 g). DIG-Piv (10.4 g) was dissolved in methanol (10 mL), then 5 M aqueous sodium hydroxide solution (60 mL) was added, and the mixture was refluxed at 70°C for 40 min. The reaction solution was extracted with dichloromethane, dehydrated over sodium sulfate, filter filtrated, and concentrated under reduced pressure. Thereto was added ethanol, and the mixture was heated with a dryer and pure water was added by small portions. When the resultant product was precipitated, the mixture was cooled to 4°C, and the resultant product DIG(6) was obtained by recrystallization. Successively, DIG-PEG-NH₂ was synthesized. To be specific, DIG(6) (260 mg) was dissolved in THF (15 mL), and potassium naphthalene (2.68 mL) was added dropwise. Thereto was added ethylene oxide (2.68 mL) and the mixture was reacted at 35°C for 24 hr. This was reprecipitated from diethyl ether and recovered by suction filtration to give DIG-PEG-OH (molecular weight 2000 Da). Successively, DIG(6)-PEG-OH (2.1 g) was dissolved in THF (24 mL), TEA (0.7 mL) was added, MsCl/THF (0.4 mL/2.7 mL) was added and the mixture was stirred in a water bath for 30 min. After stirring, the mixture was stirred at room temperature for 3 hr. This was reprecipitated in diethyl ether, and the precipitate was once recovered by suction filtration. 28% NH₃ aqueous solution (138 mL) was added and the mixture was stirred at room temperature for 4 days. This was concentrated under reduced pressure, purified by dialysis, and recovered by freeze-drying to give DIG(6)-PEG-NH₂. Successively, BLA-NCA (9.5 g) was dissolved in N,N'-dimethylformamide (DMF) (10 mL) and diluted with dichloromethane (DCM) (75 mL). On the other hand, DIG(6)-PEG-NH₂ (molecular weight 2,000) (1.0 g) synthesized above was dissolved in DMF (10 mL), and the solution was added to BLA-NCA solution. The mixed solution was subjected to polymerization for 40 hr while maintaining at 35°C. The completion of the polymerization reaction was confirmed by infrared spectroscopy (IR) analysis, the reaction mixture was added dropwise to diethyl ether (2 L) and the precipitated polymer was recovered by suction filtration, washed with diethyl ether and vacuum dried to give DIG(6)-PEG_{(2K)}-PBLA. Next, polyethylene glycol-poly((5-aminopentyl)-aspartic acid) block copolymer (DIG(6)-PEG_{(2K)}-P(Asp-AP)) was synthesized from DIG (6)-PEG_{(2K)}-PBLA. To be specific, DIG(6)-PEG_{(2K)}-PBLA (1 g) freeze-dried with benzene was dissolved in NMP (10 mL). 1,5-Diaminopentane (DAP) (8 mL) was added to DIG(6)-PEG-PBLA solution. The mixed solution was reacted for 1 hr while maintaining at 5°C. Thereafter, 20 wt% aqueous acetic acid solution (15.2 mL) was added to the reaction mixture and the mixture was dialyzed against water using a dialysis membrane (fraction molecular weight 6,000 - 8,000). The solution in the membrane was freeze-dried to give DIG(6)-PEG_{(2K)}-P(Asp-AP) (954 mg, yield 81%).

### (2) Synthesis of (Boc-Asp)-PEG(_{2K})-PAsp

First, N₃-PEG_{(2K)}-PBLA was synthesized. To be specific, THP-PEG-OH (molecular weight, 2000) (6.75 g) was dissolved in benzene, freezed and then dried under reduced pressure. This was dissolved in THF (45 mL), TEA (1.83 mL) was added, MsCl/THF (1.13 mL/22.5 mL) was further added and the mixture was reacted in a water bath for 30 min and at room temperature for 1 hr. This was reprecipitated from ether (1.3 L) and vacuum dried to recover THP-PEG-Ms. THP-PEG-Ms (6.55 g) was dissolved in DMF (44 mL), sodium azide (3.7 g) was added and the mixture was reacted at 45°C for 3 days. Pure water (30 mL) was added, and the mixture was purified by dialysis and vacuum dried to give THP-PEG-N₃. THP-PEG-N₃ (4.87 g) was dissolved in MeOH (68 mL), 1N HCl (42 mL) was added and the mixture was reacted at room temperature for 5 hr. Thereafter, an excess amount of acid was neutralized with aqueous ammonia, and the mixture was purified by dialysis and reprecipitation and dried under reduced pressure to give N₃-PEG-OH. N₃-PEG-OH (3.87 g) was dissolved in THF (36 mL), TEA (1.3 mL) was added, MsCl/THF (0.78 mL/5.4 mL) was added and the mixture was stirred in a water bath for 30 min, and then stirred at room temperature for 3 hr. This was reprecipitated from ether, and the precipitate was once recovered by suction filtration. 28% NH₃ aqueous solution (276 mL) was added and the mixture was stirred at room temperature for 4 days. This was concentrated under reduced pressure, purified by dialysis, and recovered by freeze-drying to give N₃-PEG-NH₂ (3.7 g). Successively, BLA-NCA (18.9 g) was dissolved in N,N'-dimethylformamide (DMF) (19 mL) and diluted with dichloromethane (DCM) (150 mL). On the other hand, N₃-PEG-NH₂ (molecular weight 2,000) (2.0 g) synthesized above was dissolved in DMF (20 mL), and the solution was added to BLA-NCA solution. The mixed solution was subjected to polymerization for 40 hr while maintaining at 35°C. The completion of the polymerization reaction was confirmed by infrared spectroscopy (IR) analysis, the reaction mixture was added dropwise to diethyl ether (3.8 L) and the precipitated polymer was recovered by suction filtration, washed with diethyl ether and vacuum dried to give N₃-PEG_{(2K)}-PBLA.

Next, (Boc-Asp) -PEG_{(2K)}-PBLA was synthesized from N₃-PEG_{(2K)}-PBLA. To be specific, meso-2,3-diaminosuccinic acid (234 mg) was dispersed in ethanol (50 mL), thionyl chloride (3 mL) was added dropwise under ice-cooling and the mixture was refluxed for 3 days. This was dried under reduced pressure, dissolved in water/dioxane (2 mL/5 mL), TEA (234 µL) and (Boc)₂O (214 µL) were successively added, and the mixture was reacted at 50°C overnight. This was concentrated under reduced pressure, and only a fraction of a compound in which one of the amino groups in the molecule is protected by Boc and the other is remained was extracted with silica gel column. On the other hand, 5-hexynoic acid (839 mg) was dissolved in DMF (8 mL), NHS (1.5 g) and EDC (2 g) were added, and the mixture was reacted at room temperature overnight. This was purified by extraction, concentrated under reduced pressure, and applied to silica gel column to give the object product 5-hexynoic acid N-hydroxysuccinimide ester. The thus-obtained compound of the following formula (20 mg) and N₃-PEG_{(2K)}-PBLA (500 mg) were dissolved in DSMO (25 mL) and, in the presence of 1 M aqueous copper sulfate (II) solution (60 µL) and 1 M aqueous ascorbic acid solution (60 µL), freezed at 4°C, warmed to 30°C and reacted overnight, whereby (Et₂-Boc-Asp) -PEG_{(2K)}-PBLA was synthesized, wherein Et₂-Boc-Asp was introduced into the PEG terminal. Thereafter, in the presence of 1N sodium hydroxide, the mixture was reacted 3 hours at room temperature, the carboxyl group was deprotected, and an excess amount of EDTA. 2Na was added. The mixture was left standing at room temperature overnight to remove copper ion, whereby (Boc-Asp)-PEG_{(2K)}-PBLA (438 mg) was synthesized.

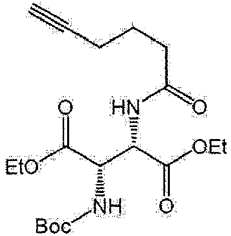

Next, Asp-PEG_{(2K)}-P(Asp) was synthesized from (Boc-Asp)-PEG_{(2K)}-PBLA. To be specific, (Boc-Asp) -PEG_{(2K)}-PBLA (100 mg) was suspended in 0.5N sodium hydroxide to hydrolyze benzyl ester at room temperature. The copolymer was dissolved, and the mixture was dialyzed against water using dialysis membrane (fraction molecular weight 6,000-8,000). The solution in the membrane was freeze-dried to give (Boc-Asp)-PEG_{(2K)}-P (Asp) (55 mg, yield 78%). As a result of confirmation by NMR spectrum, it was confirmed that Boc-Asp ligand was introduced into PEG terminal of almost all polymers (Fig. 11). Thereafter, Boc protecting group was deprotected by treating with 80% TFA to give Asp-PEG-P(Asp) (40 mg). As a result of confirmation by NMR spectrum, it was confirmed that almost all Boc groups were deprotected (Fig. 12).

### (3) Formation of PIC micelle

Glc-PEG_{(12K)}-P(Asp-AP) (50 mg) synthesized by the above-mentioned steps was dissolved in 10 mM phosphate buffer (PB, pH 7.4, 0 mM NaCl) (50 mL) to prepare 1 mg/mL Glc-PEG_{(12K)}-P(Asp-AP) solution. Similarly, (Boc-Asp)-PEG_{(2K)}-PAsp (50 mg) synthesized by the above-mentioned steps was dissolved in PB (50 mL) to prepare 1 mg/mL (Boc-Asp)-PEG_{(2K)}-PAsp solution. The above-mentioned two kinds of aqueous solutions, namely, Glu-PEG(_{12K})-P(Asp-AP) solution (4 mL) and (Boc-Asp)-PEG_{(2K)}-PAsp solution (7.0 mL) were added in 50 mL conical tube, and the mixture was stirred with vortex for 2 min (2000 rpm). Thereafter, PB solution (5.6 mL) containing a water-soluble condensing agent 1-ethyl-3-(3-dimethylaminopropyl)carbodiimide hydrochloride (EDC) (10 mg/mL) was added, and the mixture was stood overnight to crosslink the core of the polyion complex. Thereafter, polymers not involved in the micelle formation and EDC byproducts and the like were removed by using an ultrafiltration tube with a membrane having a fraction molecular weight of 100,000.

### [Experimental Example 6] Preparation of Asp-modified micelle

According to Experimental Example 5(3), Asp-PEG_{(12K)}-PAsp or PEG_{(12K)}-PAsp, and PEG_{(12K)}-P(Asp-AP) were dissolved in 10 mM phosphate buffer, mixed at a point where the electric charge ratio is balanced, and the mixture was stirred at room temperature for 2 min. 10 equivalents of EDC was added, and the mixture was stood at room temperature for 12 hr and the core of the polyion complex was crosslinked. The micelle was purified by ultrafiltration. The obtained micelles were each evaluated by dynamic light scattering measurement (Zetasizer, Malvern Instruments Ltd) to find that monodispersed particles having an average particle size of about 25 - 30 nm were obtained (Table 1).

**Table 1**

| Sample | Size (d. nm) | Pdl | Zeta potential (mV) |
|---|---|---|---|
| (2) Asp-modified micelle (Asp/m) | 29 | 0.078 | -10.5 |
| (3) Asp/m after purification | 26 | 0.082 | -9.77 |
| (4) Null micelle (Null/m) | 31 | 0.042 | 2.07 |
| (5) Null/m after purification | 30 | 0.047 | 1.27 |

These results show that even when a polymer in which Boc group is deprotected used, Asp-modified micelle can be prepared.

### [Experimental Example 7] Production of PIC micelle composed of Glc-PEG_{(12K)}-SS-P(Asp-AP) and (Boc-Asp)-PEG-PAsp

In this Experimental Example, PIC micelle containing the cleavable first block polymer modified by glucose ligand, Glc-PEG_{(12K)}-SS-P (Asp-AP) and, and the second block copolymer modified by Boc-amino group-modified aspartic acid, (Boc-Asp)-PEG_{(2K)}-PAsp, was produced.

### (1) Synthesis of Glc-PEG_{(12K)}-SS-P(Asp-AP)

First, a functional group having a disulfide bond was introduced into the terminal of polyethylene glycol (molecular weight 12,000). To be specific, polyethylene glycol having a methoxy group terminal and an aminoethyl group terminal (PEG_{(12K)}-NH₂) (molecular weight 12,000) (500 mg) was dissolved in a mixed solvent of DMF 2 mL/DCM 8 mL, and the solution was reacted with succinic anhydride (70 mg) added thereto to synthesize PEG_{(12K)}-NH-co-C₂H₄-COOH (470 mg) (yield 94%) . Introduction of the above-mentioned desired group having a carboxylic acid group to the PEG_{(12K)} terminal was confirmed by ¹H-NMR and ion exchange chromatography. Then, the obtained PEG_{(12K)}-NH-CO-C₂H₄-COOH (480 mg) was dissolved in pure water, adjusted to pH=7 with 1N NaOH, H₂N-C₂H₄-S-S-C₂H₄-NH₂•2HCl (138 mg) and EDC (117 mg) were added, and the mixture was reacted at room temperature overnight to synthesize PEG_{(12K)}-NH-CO-C₂H₄-NH-C₂H₄-S-S-C₂H₄-NH₂•HCl (478 mg) (hereinafter to be abbreviated as PEG_{(12K)}-SS-NH₂) (yield 95%). Introduction of the desired group having a disulfide bond to the PEG_{(12K)} terminal was confirmed by ¹H-NMR and ion exchange chromatography.

Next, polyethylene glycol-disulfide-poly((5-aminopentyl)-aspartic acid) block copolymer (PEG_{(12K)}-SS-P(Asp-AP)) was synthesized from the above-mentioned PEG_{(12K)}-SS-NH₂. To be specific, the above-mentioned PEG_{(12K})-SS-NH₂ (478 mg) and NCA-BLA (370 mg) were dissolved in a mixed solvent of DMF 0.4 mL/DCM 4 mL, reacted at 35°C for 2 days, and purified and recovered by reprecipitation. Then, H₂N-C₅H₁₀-NH₂ (50 equivalents) was added and the mixture was reacted at 5°C for 1 hr to polymerize (5-aminopentyl)-aspartic acid at the NH₂ side terminal of the above-mentioned PEG_{(12K)}-SS-NH₂ to give PEG_{(12K)}-SS-P(Asp-AP) (yield 89%). As a result of the measurement of ¹H-NMR, the degree of polymerization of P(Asp-AP) block was 72. It was confirmed by size-exclusion chromatography that almost monodispersed PEG_{(12K)}-SS-P (Asp-AP) was obtained.

### (2) Synthesis of (Boc-Asp) -PEG_{(2K)}-PAsp

Synthesis was performed in the same manner as in the above-mentioned Experimental Example 5, whereby (Boc-Asp)-PEG_{(2K)}-PAsp was synthesized.

### (3) Formation of PIC micelle

Glu-PEG_{(12K)}-SS-P(Asp-AP) (50 mg) synthesized by the above-mentioned steps was dissolved in 10 mM phosphate buffer (PB, pH 7.4, 0 mM NaCl) (50 mL) to prepare 1 mg/mL Glu-PEG_{(12K)}-SS-P(Asp-AP) solution. Similarly, (Boc-Asp) -PEG_{(2K)}-PAsp (50 mg) synthesized by the above-mentioned steps was dissolved in PB (50 mL) to prepare 1 mg/mL (Boc-Asp)-PEG_{(2K)}-PAsp solution. The above-mentioned two kinds of aqueous solutions, namely, Glu-PEG_{(12K)}-SS-P (Asp-AP) solution (4 mL) and (Boc-Asp) -PEG_{(2K)}-PAsp solution (7.0 mL) were added in 50 mL of conical tube, and the mixture was stirred with vortex for 2 min (2000 rpm). Thereafter, PB solution (5.6 mL) containing a water-soluble condensing agent 1-ethyl-3-(3-dimethylaminopropyl)carbodiimide hydrochloride (EDC) (10 mg/mL), and the mixture was stood overnight to crosslink the core of the polyion complex. Thereafter, polymers not involved in the micelle formation and EDC byproducts and the like were removed by using an ultrafiltration tube with a membrane having a fraction molecular weight of 100,000.

### [Experimental Example 8] Production of DIG(6)-PEG-SS-P(Asp-AP)

1,2-O-Isopropylidene-α-D-glucofranose (10.13 g) was transferred into a flask, pyridine (60 mL) was added, then dichloromethane (60 mL) was added and the mixture was completely dissolved. Thereafter, pivaloyl chloride (5.5 mL) was added dropwise, and the mixture was stirred at room temperature for 5 hr. The reaction solution was concentrated under reduced pressure, pure water was added to precipitate the resultant product, and the resultant product was recovered by filter filtration. Successively, methanol warmed to 65°C in an oil bath at 65°C was added by small portions. When completely dissolved, the power of the oil bath was turned off, and the mixture was allowed to cool to room temperature. Furthermore, the mixture was transferred to a refrigerator at 4°C and the resultant product was precipitated, and the resultant product MIG-Piv (9.25 g) was recovered by suction filtration. MIG-Piv (9.25 g) was transferred to a flask, Acetone Dimethyl Acetal (230 mL) was added and then TsOH•H₂O (292 mg) was added. This was refluxed at 75°C for 30 min, cooled to room temperature, trimethylamine (1 mL) was added dropwise, and then toluene (50 mL) was added. This was concentrated under reduced pressure, toluene (50 mL) was further added, and, similarly, concentration and addition of toluene were repeated 3 times. Thereafter, the resultant product was extracted with dichloromethane, dehydrated over sodium sulfate, suction filtered, dried under reduced pressure and recovered. This was purified by silica gel column to recover the resultant product DIG-Piv (10.4 g). DIG-Piv (10.4 g) was dissolved in methanol (10 mL), then 5 M aqueous sodium hydroxide solution (60 mL) was added, and the mixture was refluxed at 70°C for 40 min. The reaction solution was extracted with dichloromethane, dehydrated over sodium sulfate, filter filtrated, and concentrated under reduced pressure. Thereto was added ethanol, and the mixture was heated with a dryer and pure water was added by small portions. When the resultant product was precipitated, the mixture was cooled to 4°C, and the resultant product DIG(6) was obtained by recrystallization. Successively, DIG-PEG-NH₂ was synthesized. To be specific, DIG(6) (260 mg) was dissolved in THF (15 mL), and potassium naphthalene (2.68 mL) was added dropwise. Thereto was added ethylene oxide (2.68 mL) and the mixture was reacted at 35°C for 24 hr. This was reprecipitated from diethyl ether and recovered by suction filtration to give DIG(6)-PEG-OH (molecular weight 2000Da). Successively, DIG(6)-PEG-OH (2.1 g) was dissolved in THF (24 mL), TEA (0.7 mL) was added, MsCl/THF (0.4 mL/2.7 mL) was added and the mixture was stirred in a water bath for 30 min, and then stirred at room temperature for 3 hr. This was reprecipitated from diethyl ether, and the precipitate was once recovered by suction filtration. 28% NH₃ aqueous solution (138 mL) was added and the mixture was stirred at room temperature for 4 days. This was concentrated under reduced pressure, purified by dialysis, and recovered by freeze-drying to give DIG(6)-PEG-NH₂. Successively, DIG(6)-PEG-SS-NH₂ was synthesized. To be specific, DIG (6) -PEG_{(2K)}-NH₂ (300 mg) was dissolved in a mixed solvent of DMF 4 mL/DCM 16 mL, and the solution was reacted with succinic anhydride (70 mg) added thereto to synthesize DIG (6) -PEG_{(2K)}-NH-CO-C₂H₄-COOH. Introduction of the above-mentioned desired group having a carboxylic acid group to the PEG_{(2K)} terminal was confirmed by ¹H-NMR and ion exchange chromatography. Then, the obtained DIG (6) -PEG_{(2K)}-NH-CO-C₂H₄-COOH (200 mg) was dissolved in pure water, adjusted to pH=7 with 1N NaOH, H₂N-C₂H₄-S-S-C₂H₄-NH₂•2HCl (138 mg) and EDC (117 mg) were added, and the mixture was reacted at room temperature overnight to synthesize DIG(6)-PEG_{(2K)}-NH-CO-C₂H₄-NH-C₂H₄-S-S-C₂H₄-NH₂•HCl (hereinafter to be abbreviated as PEG_{(12K)}-SS-NH₂). Introduction of the desired group having a disulfide bond to the PEG_{(12K)} terminal was confirmed by ¹H-NMR and ion exchange chromatography.

Next, polyethylene glycol-disulfide-poly((5-aminopentyl)-aspartic acid) block copolymer (DIG(6)-PEG_{(2K)}-SS-P(Asp-AP)) was synthesized from the above-mentioned DIG(6)-PEG_{(2K)}-SS-NH₂•HCl. To be specific, the above-mentioned DIG (6) -PEG_{(2K)}-SS-NH₂•HCl was desalted by dialysis against 0.01% aqueous ammonia solution. DIG(6)-PEG_{(2K)}-SS-NH₂ (30 mg) and NCA-BLA (80 equivalents) were dissolved in a mixed solvent of DMF 0.4 mL/DCM 4 mL, reacted at 35°C for 2 days, and purified and recovered by reprecipitation. Then, H₂N-C₅H₁₀-NH₂ (50 equivalents) was added and the mixture was reacted at 5°C for 1 hr to polymerize (5-aminopentyl)-aspartic acid at the NH₂ side terminal of the above-mentioned DIG(6)-PEG_{(2K)}-SS-NH₂ to give DIG (6) -DIG(6)-PEG_{(2K)}-SS-P (Asp-AP) . As a result of the measurement of ¹H-NMR, the degree of polymerization of P(Asp-AP) block was 40. It was confirmed by size-exclusion chromatography that almost monodispersed DIG(6)-PEG_{(2K)}-SS-P(Asp-AP) was obtained.

### [Experimental Example 9] Production of Asp-DBCO-PEG-PAsp

According to the scheme of Fig. 13, Asp-DBCO-PEG-PAsp was produced. The reaction proceeded quantitatively, and monodispersed polymer was obtained.

### [Experimental Example 10] Evaluation of micelle uptake into GLUT1-expressing cells

### (Reagents)

### <Block copolymers>

(a) MeO-PEG₍₄₈₎-PAsp₍₇₂₎
(b) Glc(6)-PEG₍₄₈₎-PAsp₍₇₂₎: having glucose ligand at PEG terminal
(c) Asp-PEG₍₄₈₎-PAsp₍₇₂₎: having aspartic acid ligand at PEG terminal
(d) Asp-DBCO-PEG₍₄₈₎-PAsp₍₇₂₎: having aspartic acid-DBCO ligand at PEG terminal
(e) MeO-PEG-P(Asp-AP)₍₇₂₎
(f) MeO-PEG-P(Asp-AP)₍₇₂₎-Cy5

### <Micelle samples>

(A) Glc(25%)/m: micelle containing glucose ligand at proportion of 25% on surface layer
(B) Glc(25%)+Asp(25%)/m: micelle containing glucose ligand and aspartic acid ligand each at proportion of 25% on surface layer
(C) Glc(25%)+Asp-DBCO(25%)/m: micelle containing glucose ligand and aspartic acid-DBCO ligand each at proportion of 25% on surface layer

### <Preparation method of micelle>

(1) All polymers were each dissolved in 10 mM phosphate buffer (pH-7.4).
(2) To label the micelle by fluorescence, (e) and (f) were mixed at (e):(f)=6:1 (volume ratio) to prepare polycation solution.
(3) The polycation solution (2) was mixed with the polyanion solution having same number of moles of electric charge to prepare micelles. The polyanion solution used here varies depending on samples, and (a) and (b) were premixed for (A), (b) and (c) were premixed for (B), (b) and (d) were premixed for (C), such that the number of each ligand-conjugated block copolymer was 25 mol% of the total amount of the block copolymer (PEG) in the micelle surface layer. They were mixed with the polycation solution and stirred at room temperature for 2 min.
(4) A condensing agent (EDC) was added at 10 equivalents relative to the carboxyl groups in the micelle solution of (3) to immobilize the core of the micelle.
(5) (4) was purified by ultrafiltration, and the solvent was replaced with 10 mM phosphate buffer (PBS) containing 150 mM NaCl (final concentration 3 mg/mL, polymer concentration calculated from quantification of fluorescence intensity).

### <Cellular uptake assay>

(1) MDA-MB-231 cells, a GLUT1 high-expression cell line, was seeded in a 96-well plate at 3000 cells/well and incubated at 37°C for 24 hr.
(2) The above-mentioned micelle solutions (40 µL) were mixed with a medium (260 µL), respectively, 50 µL(n=6) was added per 1 well and incubated at 37°C for 30 min.
(3) A mixture of the micelle solution and the medium was removed, extracellular micelle was washed away with D-PBS(-), and a medium (50 µL) was added.
(4) The fluorescence of the micelle taken up into the cells was quantified by a fluorescence plate reader (Infinite M1000 PRO, TECAN).

As a result, all the evaluated three micelles were taken up into MDA-MB-231 cells (Fig. 14). The micelle containing glucose ligand as well as aspartic acid ligand (B) or aspartic acid-DBCO ligand (C) on the surface layer was also taken up into MDA-MB-231 cells. This suggests that the carrier can pass through BBB via GLUT1 even when the carrier contains the second molecule (the second ligand) in addition to GLUT1 ligand on the surface thereof since the first non-charged segment and the second non-charged segment have substantially the same length. However, micelles (B and C) containing aspartic acid ligand or aspartic acid-DBCO ligand in addition to glucose ligand on the surface layer thereof showed lower uptake into MDA-MB-231 cells as compared to micelle (A) containing glucose ligand alone on the surface layer. This suggests that binding of GLUT1 ligand with GLUT1 may be inhibited when the carrier contains the second molecule (the second ligand) in addition to GLUT1 ligand on the surface layer thereof, depending on the kind of the second molecule (the second ligand). Therefore, by adjusting the chain length of the first non-charged segment longer than the chain length of the second non-charged segment and placing GLUT1 ligand more outside of the carrier than the second molecule (the second ligand), inhibition of GLUT1 ligand/GLUT1 binding by the second molecule (the second ligand) is prevented during passage through BBB and efficient BBB passage can be expected.

### [Experimental Example 11] Evaluation of selective delivery capacity of micelle into brain parenchyma

### (Reagents)

### <Block copolymers>

(a) MeO-PEG₍₄₈₎-PAsp₍₇₂₎
(b) Glc(6)-PEG₍₄₈₎-PAsp₍₇₂₎: having glucose ligand at PEG terminal
(c) Asp-PEG₍₄₈₎-PAsp₍₇₂₎: having aspartic acid ligand at PEG terminal
(d) Asp-DBCO-PEG₍₄₈₎-PAsp₍₇₂₎: having aspartic acid-DBCO ligand at PEG terminal
(e) MeO-PEG-P(Asp-AP)₍₇₂₎
(f) MeO-PEG-P(Asp-AP)₍₇₂₎-Cy5

### <Micelle samples>

(A) Glc(25%)/m: micelle containing glucose ligand at proportion of 25% on surface layer
(B) Asp(25%)/m: micelle containing aspartic acid ligand at proportion of 25% on surface layer
(C) Asp-DBCO(25%)/m: micelle containing aspartic acid-DBCO ligand at proportion of 25% on surface layer

### <Preparation method of micelle>

(1) All polymers were each dissolved in 10 mM phosphate buffer (pH-7.4).
(2) To label the micelle by fluorescence, (e) and (f) were mixed at (e):(f)=6:1 (volume ratio) to prepare polycation solution.
(3) The polycation solution of (2) was mixed with polyanion solution having same number of moles of electric charge to prepare micelles. The polyanion solution used here varies depending on samples, and (a) and (b) were premixed for (A), (a) and (c) were premixed for (B), (a) and (d) were premixed for (C), such that the number of each ligand-conjugated block copolymer was 25 mol% of the total amount of the block copolymer (PEG) in the micelle surface layer. They were mixed with a polycation solution and stirred at room temperature for 2 min.
(4) A condensing agent (EDC) was added at 10 equivalents relative to the carboxyl group in the micelle solution of (3) to immobilize the core of the micelle.
(5) (4) was purified by ultrafiltration, and the solvent was replaced with 10 mM phosphate buffer (PBS) containing 150 mM NaCl (final concentration 1 mg/mL, polymer concentration calculated from quantification of fluorescence intensity).

### <Preparation of brain section>

(1) The brain was isolated from a mouse (4 days after birth) anesthetized by low temperature.
(2) (1) was embedded in agarose gel, and trimmed for each hemisphere.
(3) Using VT1200 (Leica), brain sections with thickness of 400 µm were prepared.
(4) The surrounding agarose gel was detached, the sections were placed on MilliCell (registered trade mark) and cultured for 10 days.

### <Evaluation of dispersion of micelles in brain section>

(1) The micelle solution (5 µL) prepared above was contacted with the brain section, and incubated at 37°C for 3 hr.
(2) Hoechst for nuclear staining was contacted with the section of (1).
(3) A section including the membrane of MilliCell(R) was cut out and immobilized on a culture dish with grease.
(4) The culture dish was filled with D-PBS(-), and observed with a two-photon in vivo real-time confocal laser microscope.

As a result, as compared to glucose ligand-modified micelle (A), Asp ligand-modified micelle (B) and Asp-DBCO ligand-modified micelle were significantly taken up into the parenchymal cells in the brain (Fig. 15).

The above results suggest that aspartic acid ligand promotes uptake into the parenchymal cells in the brain. A micelle modified with glucose ligand alone was not sufficiently uptaken into the cells in the brain parenchyma and many floated in the stroma. In contrast, it was clarified that aspartic acid ligand used in this experiment generally has a promoting effect on the uptake into the brain parenchyma cells. The aspartic acid ligand used in this experiment is recognized by various subtypes of glutamate receptors, and suggested to promote uptake of the carrier into various cells (e.g., neuronal cells (neuron, glial cell etc.), vascular endothelial cell) in the brain parenchyma irrespective of the kind of the subtype of the expressed glutamate receptor.

In addition, the results of Experimental Examples 10 and 11 suggest that the carrier of the present invention concurrently has a capacity to pass through blood-brain barrier and a capacity to deliver selectively to a desired site in the brain (e.g., brain vascular endothelial cell, particular tissue or cell in the brain parenchyma).

### [Industrial Applicability]

According to the present invention, a drug can be delivered more selectively to a desired site in the brain, for example, brain vascular endothelial cell and particular tissue and cell in the brain parenchyma.

This application is based on US provisional patent application No. 62/187,816 (filing date: July 2, 2015), the contents of which are incorporated in full herein.

## Claims

1. A carrier comprising a first block copolymer and a second block copolymer, wherein
the first block copolymer is a block copolymer of a first non-charged segment and a first complex-forming segment, wherein at least some proportion of the first block copolymer is modified by a first molecule, and
the second block copolymer is a block copolymer of a second non-charged segment and a second complex-forming segment, wherein at least some proportion of the second block copolymer is modified by a second molecule, and
wherein the first molecule is a GLUT1 ligand, and the second molecule is different from the first molecule.

2. The carrier according to claim 1, wherein the first complex-forming segment and the second complex-forming segment are charged segments.

3. The carrier according to claim 2, wherein the second charged segment is charged oppositely to the electric charge of the first charged segment.

4. The carrier according to claim 2 or 3, wherein at least one of the first charged segment and the second charged segment is polyamino acid.

5. The carrier according to any one of claims 1 to 4, wherein the chain length of the first non-charged segment is longer than the chain length of the second non-charged segment.

6. The carrier according to any one of claims 1 to 4, wherein the chain length of the first non-charged segment is substantially the same as the chain length of the second non-charged segment.

7. The carrier according to any one of claims 1 to 6, wherein the first block copolymer has a bond cleavable at pH 6.5 or less between the first non-charged segment and the first complex-forming segment.

8. The carrier according to any one of claims 1 to 6, wherein the first block copolymer has a bond cleavable under a reductive environment between the first non-charged segment and the first complex-forming segment.

9. The carrier according to claim 8, wherein the bond cleavable under the reductive environment is a disulfide bond.

10. The carrier according to any one of claims 1 to 9, wherein at least one of the first non-charged segment and the second non-charged segment is a polyalkylene glycol segment.

11. The carrier according to any one of claims 1 to 10, wherein an orientation of the first non-charged segment and an orientation of the second non-charged segment are the same.

12. The carrier according to any one of claims 1 to 11, wherein the first block copolymer envelops the second block copolymer.

13. The carrier according to any one of claims 1 to 12, wherein the second molecule promotes uptake into a tissue or cell in the brain parenchyma.

14. The carrier according to claim 13, wherein the second molecule has specific affinity to a particular tissue or cell in the brain parenchyma.

15. The carrier according to any one of claims 1 to 14, wherein the second molecule is aspartic acid or a derivative thereof.

16. The carrier according to any one of claims 1 to 15, wherein the GLUT1 ligand is glucose.

17. The carrier according to claim 16, wherein the glucose is connected to the first non-charged segment via a carbon at position-6.

18. The carrier according to any one of claims 1 to 17, which is a vesicle.

19. The carrier according to claim 18, wherein the vesicle has a diameter of 400 nm or less.

20. The carrier according to any one of claims 1 to 19, wherein the chain length of the first non-charged segment is longer than the chain length of the second non-charged segment, and 10 mol% or more and less than 40 mol% of the first block copolymer is modified by GLUT1 ligand.

21. The carrier according to any one of claims 1 to 19, wherein the chain length of the first non-charged segment is substantially the same as the chain length of the second non-charged segment, and 10 mol% or more and less than 40 mol% of a total of the first block copolymer and the second block copolymer is modified by GLUT1 ligand.

22. The carrier according to any one of claims 1 to 19, wherein the chain length of the first non-charged segment is longer than the chain length of the second non-charged segment, and 40 - 100 mol% of the first block copolymer is modified by GLUT1 ligand.

23. The carrier according to any one of claims 1 to 19, wherein the chain length of the first non-charged segment is substantially the same as the chain length of the second non-charged segment, and 40 - 99 mol% of a total of the first block copolymer and the second block copolymer is modified by GLUT1 ligand.

24. A composition comprising the carrier according to any one of claims 1 to 23 and a drug encapsulated in the carrier.

25. The composition according to claim 24, wherein the drug is at least one drug selected from a bioactive substance, an antibody, a nucleic acid, a biocompatible fluorescence dye and a contrast agent.

26. The composition according to claim 24 or 25, which is for delivering the drug into the brain.

27. The composition according to claim 26, which is for delivering the drug to a brain vascular endothelial cell and/or brain parenchyma.

28. The composition according to claim 26, which is for delivering the drug to a particular tissue or cell in the brain parenchyma.

29. The composition according to claim 24 or 25, which is for passing the drug through a blood-brain barrier.

30. The composition according to claim 24 or 25, which is for passing the drug through a blood-nerve barrier, a blood-retinal barrier or a blood-cerebrospinal fluid barrier.

31. The composition according to any one of claims 24 to 30, which is used for a treatment.

32. The composition according to any one of claims 24 to 30, which is used for diagnosis.

33. A method of delivering the drug into the brain of a subject, comprising administering the composition according to claim 24 or 25 to the subject.

34. The method according to claim 33, wherein the drug is delivered to a brain vascular endothelial cell and/or brain parenchyma.

35. The method according to claim 34, wherein the drug is delivered to a particular tissue or cell in the brain parenchyma.

36. A method of passing a drug through a blood-brain barrier of a subject, comprising administering the composition according to claim 24 or 25 to the subject.

37. A method of passing a drug through a blood-nerve barrier, a blood-retinal barrier or a blood-cerebrospinal fluid barrier of a subject, comprising administering the composition according to claim 24 or 25 to the subject.
